# EUROPÄISCHE PATENTANMELDUNG

(11) **EP 1 637 592 A1**
(43) Veröffentlichungstag der Anmeldung: **22.03.2006**
(21) Anmeldenummer: 05017964.7
(22) Anmeldetag: 18.12.1998
(51) Int. Cl.: C12N 5/08, C12N 5/10, A61K 35/30, A61K 48/00

(54) **Neurale Vorläuferzellen, Verfahren zu ihrer Herstellung und ihre Verwendung zur Therapie von neuralen Defekten**

(30) Priorität: 19.12.1997 DE 19756864
(62) Teilanmeldung aus: 98966817.3
(71) Anmelder: Brüstle, Oliver, Prof. Dr., 53127 Bonn (DE)
(72) Erfinder: Brüstle, Oliver, Prof. Dr., 53127 Bonn (DE)
(74) Vertreter: Grund, Martin

(57) **Zusammenfassung**

Die Erfindung betrifft isolierte und gereinigte neurale Vorläuferzellen, Verfahren zu ihrer Herstellung aus embryonalen Stammzellen in unbegrenzter Menge, die Verwendung der neuralen Vorläuferzellen zur Therapie von neuralen Defekten insbesondere bei Säugern, vorzugsweise Menschen und zur Gewinnung von Polypeptiden.

## Beschreibung

Die Erfindung betrifft isolierte und gereinigte neurale Vorläuferzellen aus embryonalen Stammzellen (ES-Zellen), Verfahren zu ihrer Herstellung in unbegrenzter Menge, die Verwendung der neuralen Vorläuferzellen zur Therapie von neuralen Defekten, insbesondere bei Säugern, vorzugsweise Menschen und zur Gewinnung von Polypeptiden.

Die Transplantation von Hirnzellen in das Nervensystem von Säugern stellt eine erfolgversprechende Methode für die Behandlung zahlreicher neurologischer Erkrankungen dar. In Tierversuchen wurden bereits zahlreiche Zellpopulationen in Gehirn und Rückenmark transplantiert (Björklund, aus: Molecular and Cellular Approaches to the Treatment of Neurological Disease, Raven Press, New York, 1993; Brüstle & McKay, Curr. Opinion Neurobiol. 6:688-695, 1996). In jüngster Zeit wurde die neurale Transplantation auch an selektierten Fällen klinisch eingesetzt, beispielsweise bei Patienten, welche an der Parkinson'schen Erkrankung leiden (Lindvall, aus: Neural transplantation in Parkinson's disease, Raven Press, New York, 1994; Olanow et al., TINS 19:102-109, 1996).

Im Gegensatz zu vielen anderen Organen zeigt das ausgereifte Nervensystem der Säuger eine außerordentlich geringe Regenerationsfähigkeit. Dies liegt im wesentlichen daran, daß die für den Aufbau des Nervensystems notwendigen Vorläuferzellen mit einigen wenigen Ausnahmen nur während der Hirnentwicklung vorhanden sind. Gerade solche unreifen Vorläuferzellen sind jedoch erforderlich, um Defekte im ausgereiften Nervensystem durch Transplantation wiederherzustellen. Dies hat zur Folge, daß als Donorgewebe für neurale Transplantate vorwiegend aus dem embryonalen Gehirn gewonnene Zellen benützt werden. So muß beispielsweise derzeit Hirngewebe von bis zu sieben menschlichen Embryonen gepoolt werden, um genügend Material für die Transplantation eines Parkinson-Patienten zu erhalten. Dies wirft nicht nur enorme ethische Probleme auf. Es ist anzunehmen, daß ein solches Verfahren auch bei Inkaufnehmen der ethischen Problematik nicht in dem Umfang umsetzbar ist, wie es für die Aufrechterhaltung einer der breiten Öffentlichkeit zugänglichen zelltherapeutischen Behandlungsstrategie erforderlich wäre.

In jüngster Zeit sind mehrere Versuche unternommen worden, die begrenzte Verfügbarkeit von embryonalen Hirnzellen von Säugern durch eine der Transplantation vorgeschaltete Vermehrung der Vorläuferzellen *in vitro* zu kompensieren. Hierbei wurden im wesentlichen zwei Strategien verfolgt. Eine Methode besteht darin, die Vorläuferzellen mit Hilfe sogenannter Onkogene zu immortalisieren. Hierbei wird ein zumeist ursprünglich aus Tumoren isoliertes Gen in das Genom der Zellen inseriert. Dieses "Tumorgen" veranlaßt die Zellen zu kontinuierlichem und in der Regel unkontrolliertem Wachstum (Lendahl & McKay, TINS 13:132-137, 1990).

Neuere und besser steuerbare Varianten dieser Methode bestehen darin, sogenannte temperatur-sensitive Mutanten zu benützen. In diesem Fall können die Zellen unter einer "permissiven" Temperatur *in vitro* vermehrt werden. Wird die nicht-permissive Temperatur entsprechend der Körpertemperatur des Empfängers gewählt, ist das Genprodukt nach Transplantation nicht mehr stabil und es kommt zum Wachstumsstop (Renfranz et al., Cell 66:713-729, 1991). Allerdings verbleibt das Onkogen in den transplantierten Zellen. Eine mögliche Schwellenaktivität oder spätere Reaktivierung kann bei diesem Verfahren nicht vollständig ausgeschlossen werden. Neurere Bemühungen gehen deshalb dahin, das Onkogen durch eine molekularbiologische Methode nach der Zellvermehrungsphase wieder aus dem Genom der Vorläuferzellen zu entfernen (Westerman & Lebouich, Proc. Natl. Acad. Sci. USA 93:8971-8976, 1996). Wie alle Zelllinien besitzen auch die Onkogen-immortalisierten Vorläuferzellen ein hohes Risiko für chromosomale Aberationen.

Eine weitere Methode zur *in vitro* Vermehrung von Vorläuferzellen vor einer Transplantation besteht darin, die aus dem Gehirn entnommenen Zellen mit Wachstumsfaktoren zu behandeln (Cattaneo & McKay, Nature 347:762-765, 1990; Reynolds & Weiss, Science 255:1707-1710, 1992; Richards et al., Proc. Natl. Acad. Sci. USA 89:8591-8595, 1992; Ray et al., Proc. Natl. Acad. Sci. USA 90:3602-3606, 1993; Kilpatrick & Bartlett, Neuron 10:255-265, 1993; Ray & Gage, J. Neurosci. 6:3548-3564, 1994; Davis & Temple, Nature 372:263-266, 1994; Vicario-Abej-n et al., Neuron 15:105-114, 1995; Gosh & Greenberg, Neuron 15:89-103, 1995; Gritti et al., J. Neurosci. 16:1091-1100, 1996). Zur Zeit kann nicht abschließend beurteilt werden, in welchem Umfang eine *in vitro* Vermehrung von Vorläuferzellen durch Wachstumsfaktorbehandlung möglich ist. Erste Transplantionsversuche mit derartig behandelten Zellen lieferten widersprüchliche Ergebnisse. Während manche Wissenschaftler eine mangelhafte Integrationsfähigkeit feststellten (Svendsen et al., Exp. Neurol. 137:376-388, 1996), scheinen andere Experimente auf eine Inkorporation solcher Zellen ins Empfängergehirn hinzuweisen (Gage et al., Proc. Natl. Acad. Sci. USA 92:11879-11883, 1995).

Zusammenfassend läßt sich bezüglich dieser Zellvermehrungsstrategien sagen, daß der mögliche Umfang einer Wachstumsfaktor-vermittelten *in vitro* Vermehrung unklar und das Verhalten derartig expandierter Zellen nach Transplantation nicht eindeutig geklärt ist. Onkogen-vermittelte Zellvermehrungsstrategien beinhalten hohe Risiken seitens des eingeführten Onkogens und etwaiger chromosomaler Aberrationen. Der schwerwiegendste Nachteil beider Strategien ist jedoch die Tatsache, daß als Anfangsmaterial Hirngewebe erforderlich ist, welches vorwiegend embryonalen Spendergehirnen entnommen wird.

Embryonale Stammzellen (ES-Zellen) bieten eine völlig neue Perspektive für die Herstellung von Donorzellen für Transplantationszwecke. Derartige Zellen wurden erstmals 1981 in der Maus beschrieben (Martin, Proc. Natl. Acad. Sci. USA 78:7634-7638, 1981; Evans & Kaufman, Nature 292:154-156, 1981). Es handelt sich um Zellen, welche beispielsweise bei der Maus aus der sog. inneren Zellmasse von etwa 3,5 Tage alten Embryonen gewonnen werden. Diese Zellen sind totipotent und können in alle Zell- und Gewebstypen ausdifferenzieren. Der augenfälligste Beweis hierfür ist die Tatasche, daß in einen anderen Embryo injizierte ES-Zellen in der Lage sind, sich an der Bildung aller Gewebe - einschließlich der Keimbahnpopulationen - zu beteiligen und sogenannte chimäre Tiere zu erzeugen (Bradley et al., Nature 309:255-256, 1984). Das einzigartige an ES-Zellen ist, daß sie sich in Anwesenheit von leukemia inhibitory factor (LIF) über viele Passagen in ihrem totipotenten Zustand halten und vermehren lassen (Smith et al., Nature 336:688-690, 1988). Dies wird heute vielfach dafür benutzt, die Zellen *in vitro* genetisch zu modifizieren, um dann nach Injektion dieser Zellen in eine Blastozyste genetisch veränderte Tiere herzustellen (Robertson et al., Nature 323:445-448, 1986). Ein weitaus weniger häufig begangener Weg ist die *in vitro* Differenzierung von ES-Zellen. Diese Technik erlaubt es u.a., die frühen Schritte der Gewebeentwicklung unter kontrollierten Bedingungen *in vitro* ablaufen zu lassen und experimentell zu untersuchen. ES-Zellen sind mittlerweile aus den verschiedensten Spezies isoliert worden, so z.B. aus Ratten (Iannaconne et al., Dev. Biol. 163:288-292, 1994), Hamstern (Doetschman et al., Dev. Biol. 127:224-227, 1988), Vögeln (Pain et al., Development 122:2339-2348, 1996), Fischen (Sun et al., Mol. Mar. Biol. Biotechno. 4:193-199, 1995), Schweinen (Wheeler, Reprod. Fertil. Dev. 6:563-568, 1994), Rindern (First et al., Reprod. Fertil. Dev. 6:553-562) und Primaten (Thomson et al., Proc. Natl. Acad. Sci. USA 92:7844-7848, 1995). Mehrere Monate nach Einreichung der prioritätsbegründenden deutschen Patentanmeldung Nr. 197 56 864.5 gelang es zwei Forschergruppen, ES Zellen bzw. ES-Zell-ähnliche Stammzellen aus menschlichem Embryonalgewebe zu isolieren (Thomson et al., Science 282: 1145-1147, 1998; Shamblott et al., Proc. Natl. Acad. Sci. USA 95: 13726-13731, 1998). Neuere Studien weisen außerdem darauf hin, daß Embryonen und damit ES-Zellen auch durch Transplantation von aus embryonalen und reifen Säugetierzellen gewonnenen Zellkernen in enukleierte Eizellen (Oocyten) generiert werden können (Campbell et al., Nature 380:64-66, 1996; Wilmut et al., Nature 385:810-813, 1997).

Während der vergangenen Jahre ist es mehreren Forschungsgruppen gelungen, ES-Zellen *in vitro* in Zellen des Nervensystem auszudifferenzieren. In der überwiegenden Zahl der Fälle wurde dies durch Zugabe von Retinsäure zu aggregierten ES-Zellen erreicht (Bain et al., Dev. Biol. 168:342-357, 1995; Strübing et al., Mech. Dev. 53:275-287, 1995; Fraichard et al., J. Cell Sei. 108:3181-3188, 1995; Finley et al., J. Neurosci. 16:1056-1065, 1996). Einige der unter diesen Bedingungen differenzierenden Zellen wiesen neuronale (Bain et al., Dev. Biol. 168:342-357, 1995; Strübing et al., Mech. Dev. 53:275-287, 1995; Fraichard et al., J. Cell Sci. 108:3181-3188, 1995; Finley et al., J. Neurosci. 16:1056-1065, 1996) und gliale (Fraichard et al., J. Cell Sci. 108:3181-3188, 1995) Eigenschaften auf. Die Retinsäure-vermittelte Induktion einer neuralen Differenzierung hat zwei wesentliche Nachteile. Zum einen erfolgt eine neurale Differenzierung nur in einem Teil der Zellen. Eine befriedigende Aufreinigung dieser Zellen war bisher nicht möglich. Zum anderen übt Retinsäure einen starken differenzierenden Einfluß auf die ES-Zellen aus. Die in Anwesenheit von Retinsäure differenzierten Neuronen und Gliazellen haben zum ganz überwiegenden Teil das Vorläuferzellstadium bereits überschritten und befinden sich vielfach in einem postmitotischen Stadium. Einer Verwendung zur Transplantation und einer weiteren Anreicherung dieser Zellen sind somit Grenzen gesetzt.

Kürzlich wurde eine alternative Methode zur Gewinnung von neuralen Vorläuferzellen aus ES-Zellen beschrieben (Okabe et al., Mech. Dev. 59:89-102, 1996). Hierbei werden die zu sog. Embryoid Bodies aggregierten ES-Zellen in serumfreiem Medium ausplattiert und über mehrere Tage kultiviert. Währed dieser Zeit kommt es zu massivem Zelltod insbesondere innerhalb der nicht-neuralen Zellen. Am Ende dieser Phase zeigen mehr als 80% der Zellen eine Expression von Nestin, ein Intermediärfilament, das typischerweise in neuralen Vorläuferzellen gebildet wird (Frederiksen & McKay, J. Neurosci. 8:1144-1151, 1988; Lendahl et al., Cell 60:585-595, 1990). Diese Vorläuferzellen sind mit Hilfe von basischem Fibroblastenwachstumsfaktor (bFGF) als Zellrasen weiter vermehrbar und differenzieren nach Entzug von bFGF in Neurone und Astrozyten aus (Okabe et al., Mech. Dev. 59:89-102, 1996). Die Expansionsfähigkeit unter bFGF Gabe ist allerdings limitiert; bereits nach wenigen Passagen kommt es zu einer zunehmenden astrozytären Differenzierung der Zellen (Okabe, aus: Current Protocols in Neuroscience, John Wiley, New York, 1997). Nach einer derartig limitierten Wachstumsphase sind innerhalb der Kulturen immer noch zahlreiche undifferenzierte embryonale und nicht-neural differenzierte Zellen vorhanden. Zellpopulationen mit derartigen Kontaminationen sind für mögliche rekonstruktive Transplantationen nicht geeignet, da undifferenzierte ES-Zellen zu Tumoren (Teratokarzinome) führen können und nicht-neural differenzierte Donor-Zellen zur Ausbildung nicht-neuraler Gewebe im Transplantat führen. Bisher ist kein Verfahren bekannt, mit dem es möglich wäre, aus ES-Zellen Zellen mit neuronalen oder glialen Eigenschaften in einer Reinheit herzustellen, die eine Transplantation ohne Tumorbildung in das Nervensystem und eine funktionelle Aktivität in vivo, wie z.B. eine Remyelinisierung oder ein Nervenzellersatz mit Normalisierung des durch den Nervenzellverlust bedingten abnormen Verhaltens erlaubt.

Die Bereitstellung ausreichender Mengen definierter neuraler Vorläuferzellen stellt nach dem gegenwärtigen Stand der Technik eines der Hauptprobleme für die Transplantation von Hirnzellen dar. Zur Zeit werden solche Voriäuferzellen aus embryonalem Hirngewebe von Säugern gewonnen, wobei beispielsweise für die Transplantation eines Parkinson-Patienten Material von bis zu sieben menschlichen Embryonen erforderlich ist. Eine solche Strategie ist ethisch äußerst problematisch und keinesfalls imstande, die Behandlung einer größeren Zahl von Parkinson-Patienten auf Dauer zu gewährleisten. Bemühungen, die entnommenen Hirnzellen vor der Transplantation *in vitro* zu vermehren, haben bislang zu keinen signifikanten Verbesserungen geführt. Onkogen-vermittelte Immortalisierungsversuche sind aufgrund der Einführung eines Onkogens in die zu transplantierenden Zellen sehr riskant. Der Umfang einer möglichen Wachstumsfaktor-vermittelten Vermehrung von Vorläuferzellen ist bislang nicht signifikant. Weiterhin herrscht Skepsis über das Inkorporationsvermögen solcher Zellen nach Transplantation (Svendsen et al., Exp. Neurol. 137:376-388, 1996).

ES-Zellen stellen eine interessante alternative Donorquelle für neurale Transplantate dar. Dies liegt daran, daß sich diese Zellen über lange Zeit in einem undifferenzierten, totipotenten Zustand zu großen Zellmengen vermehren lassen (Slack, aus: From Egg to Embryo, Cambridge University Press, Cambridge, 1991). Dabei behalten sie die Fähigkeit bei, in alle Gewebe, auch in Hirngewebe, auszudifferenzieren. Allerdings war es bisher nicht möglich, selektiv neurale Vorläuferzellen aus ES-Zellen herzustellen. Versuche, ES-Zellen mit Hilfe von Retinsäure in neurale Zellen zu differenzieren (Bain et al., Dev. Biol. 168:342-357, 1995; Strübing et al., Mech. Dev. 53:275-287, 1995; Fraichard et al., J. Cell Sci. 108:3181-3188, 1995; Finley et al., J. Neurosci. 16:1056-1065, 1996) ergaben stets gemischte Zellpopulationen, in denen neurale Zellen nur einen kleinen Teil der Zellen ausmachten. Weiter wurden bisher mit Retinsäure zwar Neurone aber keine neuralen Vorläuferzellen generiert. In einer Arbeit von Dinsmore *et al.,* Cell Transplant. 5: 131-143 (1996), wurden derartige gemischte Populationen ins Gehirn Quinolinsäure-behandelter Ratten transplantiert. Quinolinsäure ist ein bekanntes Neurotoxin, das Nervenzellen schädigt und zerstört. Nach Transplantation behielten einige der Zellen ihren neuronalen Phänotyp bei. Eine funktionelle Innervation des Empfängergehirns durch diese Zellen oder gar eine Wiederherstellung verlorengegangener Hirnfunktion wurden bei diesen Experimenten nicht beobachtet (Dinsmore et al., Cell Transplant. 5:131-143, 1996). Die von Okabe geschilderte Kultivierung plattierter Embryoid Bodies in ITSFn Medium erfolgt ohne Retinsäure und liefert bis zu 85% Zellen, welche den Vorläuferzell-Marker Nestin exprimieren (Okabe et al., Mech. Dev. 59:89-102, 1996). Allerdings ist auch diese Population nicht genügend rein, um sie für rekonstruktive Zwecke zu benützen. So bilden beispielsweise in ITSFn kultivierte ES-Zellen nach Transplantation primitive neuroepitheliale Strukturen und sogar nicht-neurales Gewebe wie z.B. Knorpel und Drüsengewebe. Eine weitere Proliferation der in ITSFn kultivierten Zellen in Anwesenheit von bFGF ist möglich. Allerdings verlieren die Zellen rasch ihre Multipoteriz und differenzieren bereits nach wenigen Passagen überwiegend in Astrozyten aus. Die nicht neural differenzierten Zellen konnten während dieser kurzen Proliferationsphase bisher nicht von den neuralen Vorläuferzellen abgetrennt werden. Ein weiterer schwerwiegender Nachteil dieses Systems ist, daß es keine effiziente Produktion von Oligodendrozyten erlaubt. So fanden Okabe et al. nach Wachstumsfaktorentzug-vermittelter Differenzierung allein keine Oligodendrozyten, und selbst nach zusätzlicher Gabe des Hormons T3 ließ sich nur in 1-2% der Zellen eine Expression oligodendroglialer Antigene nachweisen (Okabe et al., Mech. Dev. 59:89-102, 1996). Was die neuronale Differenzierung anbelangt, wurden in den von Okabe et al. durchgeführten Arbeiten keine Neurone beschrieben, welche Tyrosinhydroxylase, Cholinacetyltranferase oder Serotonin produzieren, Stoffe, die für die Signalübertragung in Nervenzellen von großer Bedeutung sind. Weiter wurden in diesen Arbeiten keine neuralen Zellen erhalten, welche Peripherin produzieren. Peripherin wird typischerweise von peripheren Neuronen und Neuronen in Hirnstamm und Rückenmark produziert.

Der Erfindung liegt daher die Aufgabe zugrunde, isolierte, gereinigte nicht-tumorigene Vorläuferzellen mit neuronalen oder glialen Eigenschaften aus embryonalen Stammzellen (ES-Zellen), insbesondere gereinigte Neurone und Gliazellen, sowie Verfahren zur Herstellung der Vorläuferzellen in praktisch unbegrenzter Menge bereitzustellen. Mit Hilfe dieser gereinigten Vorläuferzellen gelingt eine Transplantation ohne Tumorbildung in das Nervensystem und eine funktionelle Aktivität in vivo, wie z.B. eine Remyelinisierung oder ein Nervenzellersatz mit Normalisierung des durch den Nervenzellverlust bedingten abnormen Verhaltens, und eine Verbesserung der Therapie neuraler Defekte. Die Lösung dieser Aufgaben ergibt sich aus den Patentansprüchen, der nachfolgenden Beschreibung und den Abbildungen.

### Definitionen

Der hier verwendete Begriff *Astrozyt* bedeutet eine Gliazelle des Nervensystems, über deren Funtion noch wenig bekannt ist. Die Fortsätze dieser Zellen bilden einen Teil der sog. Bluthirnschranke, ein Trennsystem zwischen dem Blutkreislauf und den hirneigenen Flüssigkeiten. Der hier verwendete Begriff *autolog* bedeutet, daß die Zellen vom selben Individuum stammen. Der hier verwendete Begriff *bFGF* bedeutet "Basic Fibroblast Growth Factor". bFGF ist ein Wachstumsfaktor, der u.a. neurale Vorläuferzellen zur Teilung (Proliferation) anregt. Der hier verwendete Begriff CNTF bedeutet "Ciliary Neurotrophic Factor". Der hier verwendete Begriff *DMEM*/*F12* bedeutet Dulbecco's Modified Eagle Medium/Nutritient Mix F12 (1:1). DMEM/F12 ist ein kommerziell erhältliches, für die serumfreie Zellkultur verwendetes Medium und z.B. von Life Technologies, Nr. 11320 erhältlich. Der hier verwendete Begriff *EGF* bedeutet "Epidermal Growth Factor". EGF ist ein Wachstumsfaktor, der u.a. neurale Vorläuferzellen zur Teilung (Proliferation) anregt. Der hier verwendete Begriff *Embryoid Bodies* bedeutet im Überstand schwimmende Zellaggregate, die von in Bakterienkulturschalen kultivierten ES-Zellen gebildet werden. Diese Zellaggregate zeigen Ähnlichkeit zu frühen Embryonen und bilden eine Vielzahl verschiedener Gewebe und werden deshalb als Embryoid Bodies bezeichnet. Der hier verwendete Begriff *ES-Zellen* bedeutet Embryonale Stammzellen. ES-Zellen können z.B. aus sehr frühen Embryonen von Säugern im Blastozystenstadium gewonnenen werden. Es handelt sich um totipotente Zellen, welche über viele Passagen in einem undifferenzierten Zustand gehalten werden können und die Fähigkeit besitzen, in alle Gewebe und Zelltypen auszudifferenzieren. ES-Zellen können auch durch Proliferation von Oocyten nach einer Kerntransplantation erhalten werden. Der Begriff ES-Zellen bedeutet ferner ES-Zell-ähnliche Zellen, die aus embryonalen Keimzellen (embryonic germ cells) erhalten werden. Der hier verwendete Begriff *Feeder-Zellen* bedeutet eine Zellpopulation, welche das Wachstum einer zu züchtenden Zellpopulation unterstützt. Beispielsweise werden ES-Zellen vielfach auf einem aus embryonalen Fibroblasten bestehenden Feeder-Zellrasen gezüchtet. Die hier verwendeten Begriffe *ITSF*_{*11*} und *N3FL* sind Medien, die vom DMEM/F12-Medium abgewandelt sind (Okabe et al., Mech. Dev. 59:89-102, 1996). Der hier verwendete Begriff *LIF* bedeutet "Leukemia inhibitory factor". LIF ist ein Faktor, welcher die Differenzierung von ES-Zellen verhindert. Der hier verwendete Begriff *neurale Vorläuferzellen* bedeutet unreife Zellen, welche die Fähigkeit haben, reife Zellen des Nervensystems, z.B. Neurone (Nervenzellen) und Glia (Astrozyten und Oligodendrozyten) zu bilden. Der hier verwendete Begriff *neurale Sphäroide* bedeutet durch Proliferation neuraler Vorläuferzellen in serumfreien Wachstumfaktor-haltigem Medium in unbeschichteten Zellkulturschalen erhaltene Zellaggregate. Der hier verwendete Begriff *Oligodendrozyt* bedeutet eine Zelle des Nervensystems, die den sog. Gliazellen untergeordnet ist. Wichtigste bekannte Funktion dieser Zellen ist die elektrische Isolierung von Nervenzellfortsätzen (Axonen). Dabei werden die Axone von einer von den Oligodendrozyten gebildeten Hülle (Myelin) umhüllt. Schäden in der Myelinisierung führen zu sog. demyelinisierenden Erkrankungen. Eine der bekanntesten demyelinisierenden Erkrankungen ist die multiple Sklerose (MS). Der hier verwendete Begriff *PDGF* bedeutet "Platelet-Derived Growth Factor". PDGF ist ein Wachstumsfaktor, der u.a. in Kombination mit anderen Wachstumfaktoren neurale Vorläuferzellen zur Teilung (Proliferation) anregt. PDGF kommt in Form der Dimere AA, AB und BB vor und wird als *PDGF-AA, PDGF-AB* oder *PDGF-BB* bezeichnet. Der hier verwendete Begriff *toti-, pluri-, multi- und bipotente Zellen* bedeutet Vorläuferzellen, welche in alle (totipotent), viele verschiedene (pluri- oder multipotent) oder zwei (bipotent) reife Zelltypen ausdifferenzieren können. In der Neurobiologie werden bipotente Zellen oft als Synonym für solche Vorläuferzellen verwendet, die noch in Astrozyten und Oligodendrozyten ausdifferenzieren können.

Somit betrifft die Erfindung isolierte, nach Transplantation in das Nervensystem nicht-tumorigene gereinigte Vorläuferzellen aus embryonalen Stammzellen (ES-Zellen) mit neuronalen oder glialen Eigenschaften, die vorzugsweise höchstens etwa 15% primitive embryonale und nicht-neurale Zellen enthalten. Bevorzugt sind Vorläuferzellen in Form eines Zellrasens oder Sphäroiden. Besonders bevorzugt sind Vorläuferzellen mit neuronalen, astrozytären und/oder oligodendroglialen Eigenschaften. Ferner bevorzugt sind Vorläuferzellen, die nach Proliferation von Oocyten, die durch Kerntransplantation hergestellt wurden, erhalten werden. Ferner bevorzugt sind Vorläuferzellen, die aus embryonalen Stammzellen erhalten werden, die ihrerseits aus embryonalen Keimzellen (embryonic germ cells) erhalten werden. Besonders bevorzugt sind Vorläuferzellen. die von Säuger-ES-Zellen oder Säuger-Oocyten erhalten werden. Insbesondere bevorzugt sind Vorläuferzellen der Maus, Ratte, Hamster, Schaf, Schwein, Rind, Primaten oder Mensch. Ferner besonders bevorzugt sind Vorläuferzellen, die gentechnisch modifiziert sind. Besonders bevorzugt sind Vorläuferzellen in tiefgefrorener Form. Ferner bevorzugt sind Zellbibliotheken, umfassend autologe und nicht autologe Vorläuferzellen.

Die Erfindung betrifft ferner ein Verfahren zur Herstellung der gereinigten Vorläuferzellen mit neuronalen oder glialen Eigenschaften, umfassend die folgenden Schritte: a) proliferieren von ES-Zellen, b) kultivieren der ES-Zellen aus Schritt a) zu neuralen Vorläuferzellen, c) proliferieren der neuralen Vorläuferzellen in einem Wachstumsfaktor-haltigen serumfreien Medium, d) proliferieren der neuralen Vorläuferzellen aus Schritt c) in einem weiteren Wachstumsfaktor-haltigen serumfreien Medium und isolieren der gereinigten Vorläuferzellen und e) proliferieren der Vorläuferzellen aus Schritt d) in einem weiteren Wachstumsfaktor-haltigen serumfreien Medium und isolieren der gereinigten Vorläuferzellen mit neuronalen oder glialen Eigenschaften. Bevorzugt ist eine Proliferation der ES-Zellen in Schritt a) zu Zellaggregaten, insbesondere Embryoid Bodies. Ferner bevorzugt ist ein Verfahren, wobei das Wachstumsfaktor-haltige serumfreie Medium in Schritt c) bFGF umfaßt. Ferner bevorzugt ist ein Verfahren, wobei das Wachstumsfaktor-haltige serumfreie Medium in Schritt d) bFGF und EGF umfaßt. Ferner bevorzugt ist ein Verfahren, wobei das Wachstumsfaktor-haltige serumfreie Medium in Schritt e) bFGF und PDGF umfaßt. Ferner bevorzugt ist ein Verfahren in dem die gereinigte neurale Vorläuferzelle in einem Injektionsmedium aufgenommen wird.

Die Erfindung betrifft ferner ein Verfahren zur Herstellung der gereinigten Vorläuferzellen mit neuronalen oder glialen Eigenschaften, umfassend die folgenden Schritte: a') proliferieren von ES-Zellen, b') kultivieren der ES-Zellen aus Schritt a') zu neuralen Vorläuferzellen, c') proliferieren der neuralen Vorläuferzellen in einem Wachstumsfaktor-haltigen serumfreien Medium, d') proliferieren der Vorläuferzellen aus Schritt c') in einem weiteren Wachstumsfaktor-haltigen serumfreien Medium zu neuralen Sphäroiden und isolieren der neuralen Sphäroide und e') proliferieren der neuralen Sphäroide aus Schritt d') in einem Wachstumsfaktor-haltigen serumfreien Medium bis zur Ausbildung eines aus glialen Vorläuferzellen bestehenden Zellrasens und isolieren der gereinigten Vorläuferzellen. Bevorzugt ist eine Proliferation der ES-Zellen in Schritt a') zu Zellaggregaten, insbesondere Embryoid Bodies. Bevorzugt ist ferner ein Verfahren f), wobei die in Schritt e') gewonnenen glialen Vorläuferzellen durch die Zugabe einzelner Faktoren in eine astrozytäre oder oligodendrogliale Richtung gesteuert und isoliert werden. Besonders bevorzugt ist ein Verfahren, wobei das Wachstumsfaktor-haltige serumfreie Medium in Schritt c') bFGF umfaßt. Ferner besonders bevorzugt ist ein Verfahren, wobei das Wachstumsfaktor-haltige serumfreie Medium in den Schritten d'), e') und f) die Wachstumsfaktoren bFGF und EGF einzeln oder in Kombination umfaßt. Ferner besonders bevorzugt ist ein Verfahren, wobei in Schritt f) für die Induktion einer astrozytären Differenzierung der "ciliary neurotrophic factor" (CNTF) und für die Induktion und Ausreifung oligodendroglialer Zellen das Schilddrüsenhormon T3 benützt werden. Ferner bevorzugt ist ein Verfahren in dem die gereinigte neurale Vorläuferzelle in einem Injektionsmedium aufgenommen wird.

Die erfindungsgemäßen neuralen Vorläuferzellen können als Arbeitsmittel (Arzneimittel) in der Medizin verwendet werden. Bevorzugt ist die Verwendung der gereinigten neuralen Vorläuferzellen zur Herstellung eines Arbeitsmittels (Arzneimittels) zur Therapie von neuralen Defekten. Besonders bevorzugt ist die Verwendung der gereinigten neuralen Vorläuferzellen zur Rekonstitution von neuronalen Zellen oder zur Remyelinisierung demyelinisierter Nervenzellen insbesondere demyelinisierter Bereiche des Nervensystems mittels Zelltransplantation ins Nervensystem.

Insbesondere bevorzugt ist die Rekonstitution neuronaler Zellen, welche im Rahmen traumatischer, ischämischer, degenerativer, genetischer (anlagebedingter), hypoxischer, metabolischer, infektiöser, neoplastischer oder toxischer Schädigungen des Nervensystems in ihrer Funktion gestört oder ausgefallen sind. Bevorzugte Schädigungen des Nervensystems, auf die dieses Verfahren angewandt werden kann, umfassen unter anderen traumatische Hirn- und Rückenmarksverletzungen, ischämische und hämorrhagische Infarkte von Abschnitten des Nervensystems, M. Parkinson, M. Huntington, M. Alzheimer, anlagebedingte Atrophien insbesondere des Kleinhirns und des Hirnstammes, Motoneuronerkrankungen und spinale Formen der Muskelatrophie. Weiter bevorzugt ist die Rekonstitution neuronaler Zellen, welche im Rahmen des physiologischen Alterungsprozesses in ihrer Funktion gestört oder ausgefallen sind. Ferner insbesondere bevorzugt ist die Remyelinisierung demyelinisierter Hirnareale. Bevorzugte Schädigungen des Nervensystems, auf die die Transplantation der verfahrensgemäß gewonnenen glialen Vorläuferzellen als remyelinisierende Maßnahme angewandt werden können, umfassen unter anderen die multiple Sklerose (MS), die Adrenoleukodystrophie sowie die Pelizaeus-Merzbacher'sche Erkrankung.

Ferner bevorzugt ist die Verwendung der neuralen Vorläuferzellen für zellvermittelten Gentransfer in das Nervensystem. Bevorzugte Schädigungen des Nervensystems, auf die die Transplantation der verfahrensgemäß gewonnenen glialen Vorläuferzellen in Form eines zellvermittelten Gentransfers anwendbar sind, umfassen unter anderen insbesondere auf angeborenen Enzymdefekten beruhende metabolische Erkrankungen sowie Neoplasien innerhalb des Nervensystems. Die erfindungsgemäßen Vorläuferzellen können ferner für die *in vitro* Produktion von klinisch und gewerblich nutzbaren Faktoren z.B. Polypeptiden benützt werden.

Die Erfindung wird durch Abbildungen weiter erläutert.

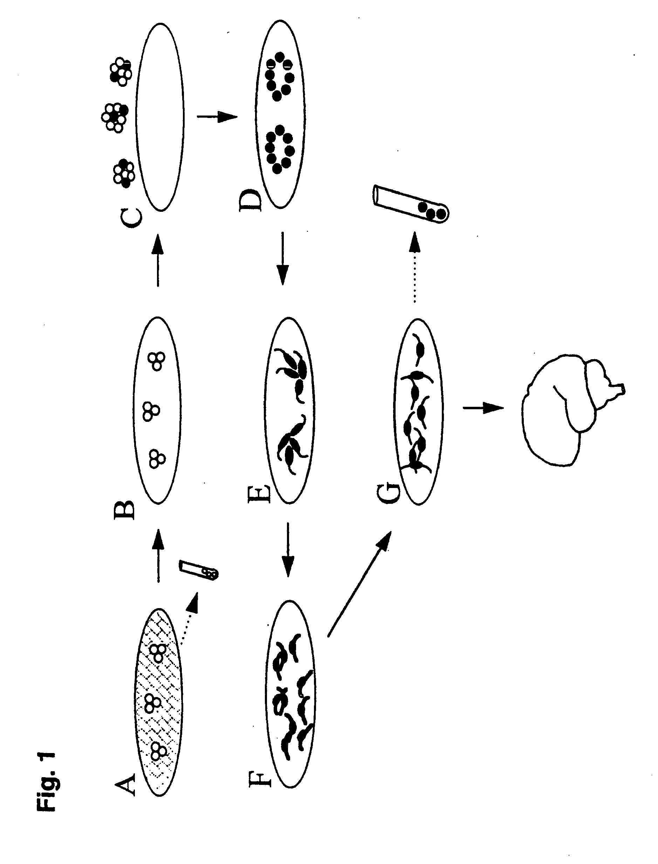

Abbildung 1 ist eine schematische Darstellung (ein Fließschema) der Gewinnung neuraler Vorläuferzellen aus ES-Zellen. A zeigt ES-Zellen (leere Kreise) auf einem Feeder-Zellrasen embryonaler Fibroblasten (kleine Rechtecke). Im Anschluß an eine Proliferation können die ES-Zellen weiterverarbeitet oder in flüssigem Stickstoff tiefgefroren werden. B zeigt ES-Zellen in Gelatine-beschichteten Feederzell-freien Zellkulturschalen. C zeigt Embryoid Bodies, in denen eine beginnende Differenzierung u.a. in neurale Zellen (schwarze Kreise) stattfindet. D zeigt plattierte Embryoid Bodies in einer Zellkulturschale, die ITSFn Medium enthält. Dieses serumfreie Medium begünstigt das Überleben neuraler Zellen (schwarze Kreise). E zeigt proliferierte neurale Vorläuferzellen, die in einem Wachstumsfaktor-haltigen serumfreien Medium (sog. N3FL Medium) in Anwesenheit von bFGF weiter proliferieren. F zeigt proliferierte neurale Vorläuferzellen, die in einem weiteren Wachstumsfaktor-haltigen serumfreien Medium (sog. N3EFL Medium) in Anwesenheit von bFGF und EGF weiter proliferieren. G zeigt proliferierte neurale Vorläuferzellen, die in einem weiteren Wachstumsfaktor-haltigen serumfreien Medium (sog. N2FP Medium) in Anwesenheit von bFGF und PDGF weiter proliferieren. Nach 2 Passagen in N2FP Medium können die Zellen für remyelinisierende Transplantate verwendet oder für eine spätere Verwendung in serumfreiem Gefriermedium tiefgefroren werden.

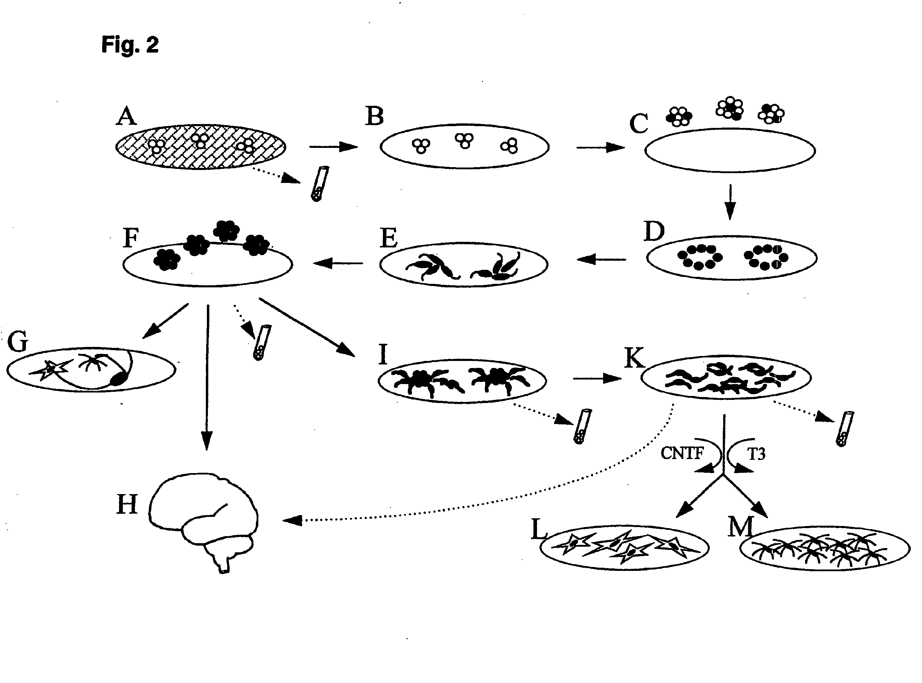

Abbildung 2 ist eine schematische Darstellung (ein Fließschema) der Gewinnung neuraler Sphäroide und der daraus abgeleiteten Zellpopulationen aus ES-Zellen. A bis E sind identisch mit Abbildung 1. F zeigt aus N3FL Kulturen in unbeschichteten Zellkulturschalen etablierte neurale Sphäroide, die in einem weiteren Wachstumsfaktor-haltigen serumfreien Medium (sog. N2EF Medium) in Anwesenheit von bFGF und EGF proliferieren und auch kryokonserviert werden können. G zeigt die *in vitro* Differenzierung solcher Sphäroide durch Wachstumsfaktorentzug, wobei reife Neurone, Astrozyten und Oligodendrozyten entstehen. H zeigt die Transplantation solcher Sphäroide in das Nervensystem. I zeigt die Entstehung glialer Vorläuferzellpopulationen in Form sog. Touch-down Kulturen. Hierbei werden die in F erhaltenen Sphäroide in Anwesenheit von Wachstumsfaktoren solange gezüchtet, bis sie beginnen, sich an unbeschichtete Zellkulturschalen anzuheften und gliale Vorläuferzellen aus den Sphäroiden auf die Zellkulturschale auswandern. K zeigt, wie nach Entfernen der adhärierten Sphäroide die auf die Zellkulturschale ausgewanderten Vorläuferzellen in Anwesenheit von Wachstumsfaktoren weiter proliferiert werden. Die in I und K erzeugten Zellen können kryokonserviert werden. Ferner ist es möglich, die in K gewonnenen glialen Vorläuferzellen ins Nervensystem zu transplantieren. L und M zeigen, wie durch Zugabe von CNTF oder T3 und konsekutivem Wachstumsfaktorentzug eine astrozytäre (L) oder oligodendrogliale (M) Differenzierung der aus ES Zellen gewonnenen glialen Vorläuferzellen induziert werden kann.

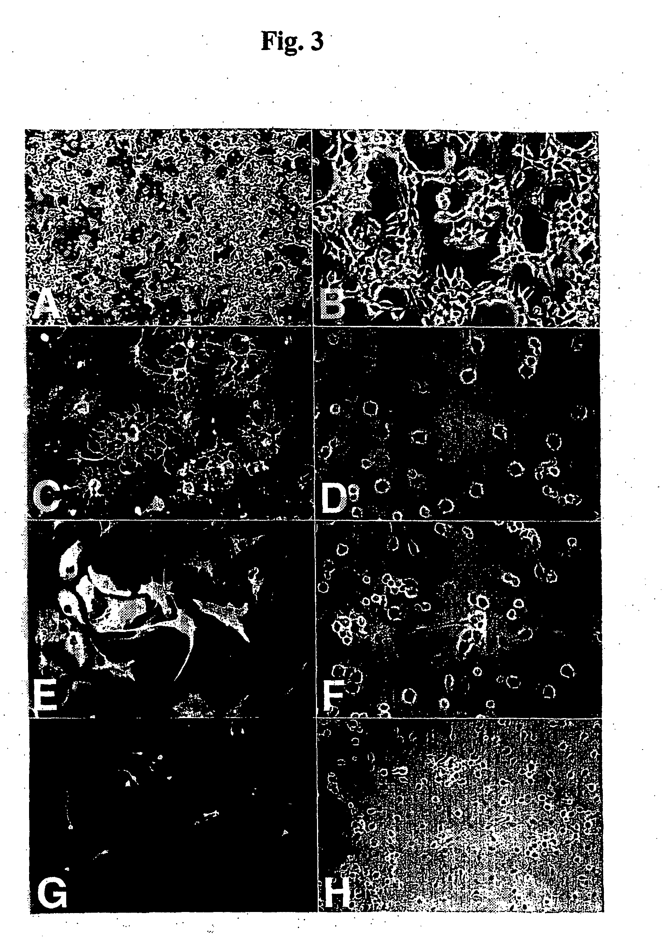

Abbildung 3 zeigt Zellkulturen von neuralen Vorläuferzellen nach *in vitro* Proliferation und Differenzierung von ES-Zellen. A und B zeigt von aus J1-ES-Zellen abgeleitete neurale Vorläuferzellen während der Proliferation in N2FP Medium in Phasenkontrast-Aufnahmen. C und D zeigt eine oligodendrogliale Differenzierung von N2FP Kulturen 4 Tage nach Entzug der Wachstumsfaktoren bFGF und PDGF. Zahlreiche Zellen exprimieren das oligodendrogliale Markerantigen 04. C ist eine Immunfluoreszenzaufnahme, D ist die korrespondierende Phasenkontrastaufnahme. E und F zeigt eine astrozytäre Differenzierung von N2FP Kulturen. Vier Tage nach Wachstumsfaktorentzug lassen sich zahlreiche GFAP-positive Astrozyten nachweisen. E ist eine Immunfluoreszenzaufnahme, F ist die korrespondierende Phasenkontrastaufnahme. G und H zeigt eine neuronale Differenzierung in N2FP Kulturen. Zusätzlich zu oligodendroglialen und astrozytären Zellen lassen sich nach Wachstumsfaktorentzug Neurone nachweisen. Diese weisen lange Fortsätze auf und zeigen eine deutliche Expression des neuronalen Markerantigens β-III-Tubulin. G ist eine Immunfluoreszenzaufnahme, H ist die korrespondierende Phasenkontrastaufnahme.

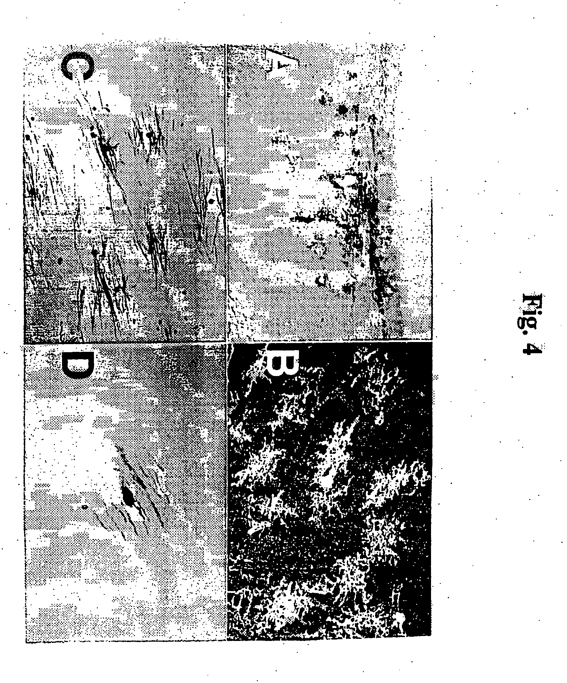

Abbildung 4 zeigt Vibratomschnitte eines Empfängergehirns nach Transplantation von aus ES-Zellen gewonnenen glialen Vorläuferzellen. Die glialen Vorläuferzellen differenzieren nach Transplantation in fetales Rattengehirn in Oligo- und Astroglia aus. Die aus ES-Zellen hergestellten Oligodendrozyten myelinisieren das Empfängergehirn. (A) zeigt von den transplantierten Zellen abstammende Astrozyten, die nach intraventrikulärer Transplantation in ein fetales Rattengehirn in das Hirnparenchym einwandern. Für dieses Experiment wurden N2FP Kulturen als Einzelzellsuspension in das Ventrikelsystem 17 Tage alter Rattenembryonen injiziert. Die Empfängertiere wurden drei Wochen nach der Geburt analysiert. Für den Nachweis der Donorzellen wurde ein Antikörper gegen das Maus-spezifische gliale Antigen M2 verwendet. Am oberen Bildrand ist der III. Ventrikel zu erkennen. (B) zeigt Inkorporation von aus ES-Zellen abgeleiteter Glia in die Großhirnrinde einer neugeborenen Ratte. Die Zellen waren am Embryonaltag 16 in das Ventrikelsystem des Empfängers implantiert worden. Die Darstellung erfolgte über eine Immunfluoreszenzuntersuchung mit einem Antikörper gegen das Maus-spezifische Antigen M6. Die Zellen weisen eine charakteristische astrozytäre Morphologie auf. (C) zeigt Inkorporation von aus ES-Zellen abgeleiteten Oligodendrozyten in die unteren Vierhügel *(colliculus inferior)* einer 22 Tage alten myelindefizienten Ratte, welche am Embryonaltag 17 eine intraventrikuläre Injektion einer N2FP Kultur erhalten hatte. Die Donorzellen sind mittels einer DNA *in situ* Hybridisierung mit einem DNA-Sondenmolekül gegen Maus-Satelliten-DNA identifiziert worden (schwarz gefärbte Zellkerne). Die inkorporierten Zellen haben begonnen, das Empfängergehirn zu myelinisieren. Da myelindefiziente Ratten kein immunreaktives PLP bilden, konnte die Myelinisierung immunhistochemisch mit einem Antikörper gegen PLP nachgewiesen werden (schwarze Fortsätze). (D) zeigt eine Nahaufnahme eines aus ES-Zellen abgeleiteten myelinisierenden Oligodendrozyten nach Inkorporation in den Hypothalamus eines 22 Tage alten Empfängertieres. Man erkennt deutlich den hybridisierten Kern und die PLP-positiven Fortsätze in typischer paralleler Anordnung.

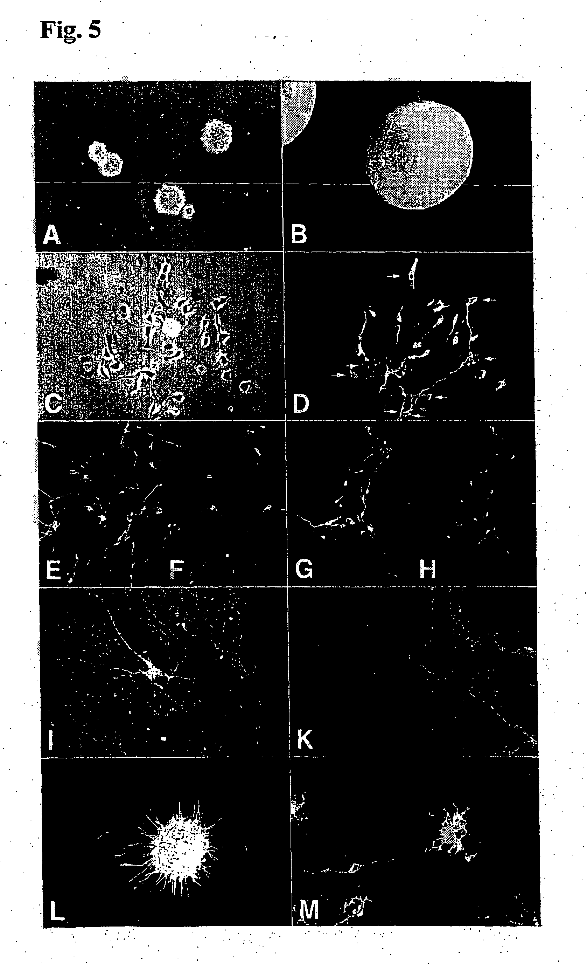

Abbildung 5 zeigt Kulturen von aus ES Zellen gewonnenen neuralen Sphäroiden und aus ihnen gewonnene Zellpopulationen. A zeigt 5 Tage alte, aus der ES Zellinie J1 gewonnene neurale Sphäroide wie sie z.B. für die Transplantation ins Nervensystem verwendet werden können. B zeigt zwei Wochen alte, aus J1-ES-Zellen gewonnene neurale Sphäroide, gefärbt mit einem Antikörper gegen das typischerweise in neuralen Vorläuferzellen exprimierte Intermediärfilament Nestin. Zu diesem Zeitpunkt haben die Sphäroide eine makroskopisch sichtbare Größe erreicht (Immunfluoreszenzaufnahme). C und D zeigen 5 Tage alte, aus der ES Zellinie CJ7 gewonnene neurale Sphäroide, welche in mit Polyornithin und Fibronektin beschichteten Zellkulturschalen ausgesät und für weitere 5 Tage ohne Wachstumsfaktoren kultiviert worden waren. Unter diesen Bedingungen desintegrieren die Sphäroide und differenzieren in neurale Zellen aus. Gezeigt ist in D eine Doppelimmunfluoreszenz-Untersuchung mit Antikörpern gegen das neuronale Antigen β-III-Tubulin (helleres Signal) und das für neurale Vorläuferzellen typische Intermediärfilament Nestin (dunkleres Signal, Pfeile). Der Vergleich mit der korrespondierenden Phasenkontrast-Aufnahme (C) läßt erkennen, daß jede der gezeigten Zellen zumindest einen dieser neuralen Marker exprimiert. E und F zeigt eine für 7 Tage auf Polyornithin und Fibronektin in Abwesenheit von Wachstumsfaktoren differenzierte, aus der ES Zellinie J1 gewonnene 5 Tage alte neurale Sphäroid-Kultur, gefärbt mit Antikörpern gegen den neuronalen Marker Microtubule-Associated Protein 2 (MAP2; E) und den Neurotransmitter GABA (F) (Immunfluoreszenz-Aufnahmen). Es ist erkenntlich, daß zahlreiche Neurone den Neurotransmitter GABA exprimieren. Zellpopulationen, welche reich an GABAergen Zellen sind, können für die Transplantation von M. Huntington-Patienten verwendet werden. G und H zeigen Zellen derselben Kultur, gefärbt mit Antikörpern gegen den neuronalen Marker Microtubule-Associated Protein 2 (MAP2; G) und den Neurotransmitter Glutamat (Immunfluoreszenz-Aufnahmen). Es ist erkenntlich, daß zahlreiche Neurone den Neurotransmitter Glutamat exprimieren. 1 zeigt eine für zwei Wochen auf Polyornithin und Fibronektin in Abwesenheit von Wachstumsfaktoren differenzierte, aus der ES Zellinie J1 gewonnene 5 Tage alte neurale Sphäroid-Kultur, doppelgefärbt mit Antikörpern gegen die neuronalen Antigene β-III-Tubulin (filamentäre Färbung in Zellfortsätzen) und Synapsin (punktförmiges Signal, den Fortsätzen anhaftend). Um die Ausreifung der Neurone zu unterstützen, wurde während der letzten 5 Tage der Differenzierung das Neurotrophin Brain-Derived Neurotrophic Factor (BDNF) zugegeben (20 ng/ml). Es ist erkenntlich, daß die aus den neuralen Sphäroiden gewonnenen Neurone sehr reife Morphologien ausbilden und eine Expression von für die neuronale Signalübertragung wichtigen synaptischen Proteinen aufweisen. K zeigt eine auf Polyornithin und Fibronektin in Abwesenheit von Wachstumsfaktoren differenzierte, aus der ES Zellinie J1 gewonnene 5 Tage alte neurale Sphäroid-Kultur, gefärbt mit einem Antikörper gegen Tyrosinhydroxylase. Tyrosinhydroxylase-positive Neurone werden z.B. für die Transplantation von Parkinson-Patienten verwendet. Diese Immunfluoreszenz-Aufnahme verdeutlicht, daß zahlreiche der aus den ES Zellen gewonnenen Neurone dieses Enzym bilden. L zeigt ein für eine Woche auf Polyornithin und Fibronektin in Abwesenheit von Wachstumsfaktoren differenziertes, aus der ES Zellinie J 1 gewonnenes 11 Tage altes neurales Sphäroid, gefärbt mit einem Antikörper gegen das astrozytäre Antigen Glial Fibrillary Acidic Protein (GFAP). Diese Immunfluoreszenz-Aufnahme verdeutlicht, daß die aus ES Zellen hergestellten neuralen Sphäroide auch Astrozyten generieren. M zeigt eine für 5 Tage auf Polyornithin und Fibronektin in Abwesenheit von Wachstumsfaktoren differenzierte, aus der ES Zellinie J1 gewonnene 5 Tage alte neurale Sphäroid-Kultur, gefärbt mit einem Antikörper gegen den oligodendroglialen Marker 04. Diese Immunfluoreszenz-Aufnahme verdeutlicht, daß die aus ES Zellen hergestellten neuralen Sphäroide auch Oligodendrozyten generieren.

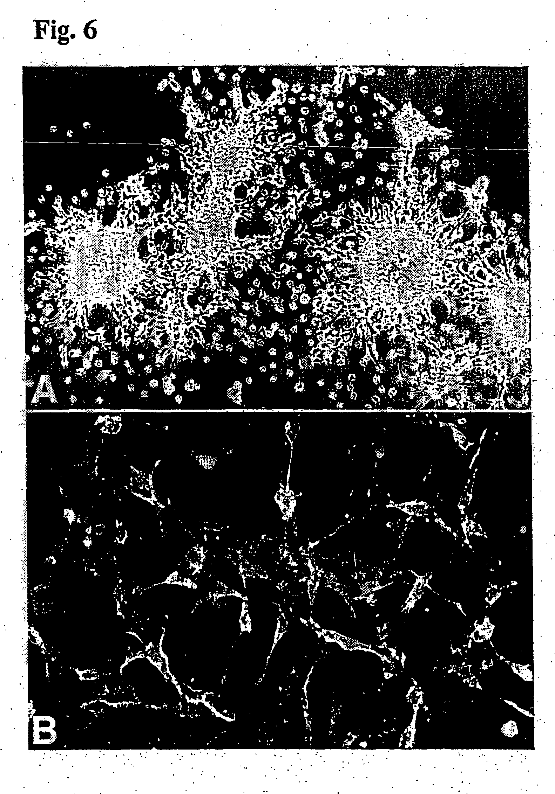

Abbildung 6 zeigt aus neuralen Sphäroiden gewonnene gliale Vorläuferzellen. A zeigt ein typisches Beispiel einer in Entstehung begriffenen sog. Touch-down Kultur. Aus der ES-Zellinie J 1 gewonnene neurale Sphäroide adhärieren an unbeschichtete Zellkulturschalen, und es kommt zum Auswandern zahlreicher glialer Zellen aus den Sphäroiden. Die adhärierten Sphäroide lassen sich durch Schütteln leicht wieder von der Zellkulturschale lösen, wobei ein reiner glialer Vorläuferzellrasen zurückbleibt. B zeigt eine Immunfluoreszenz-Untersuchung einer derartig aus der ES Zellinie J1 gewonnenen glialen Vorläuferzell-Population mit einem Antikörper gegen das neurale Antigen A2B5. Es ist erkenntlich, daß die gewonnenen glialen Vorläuferzellen dieses neurale Antigen exprimieren.

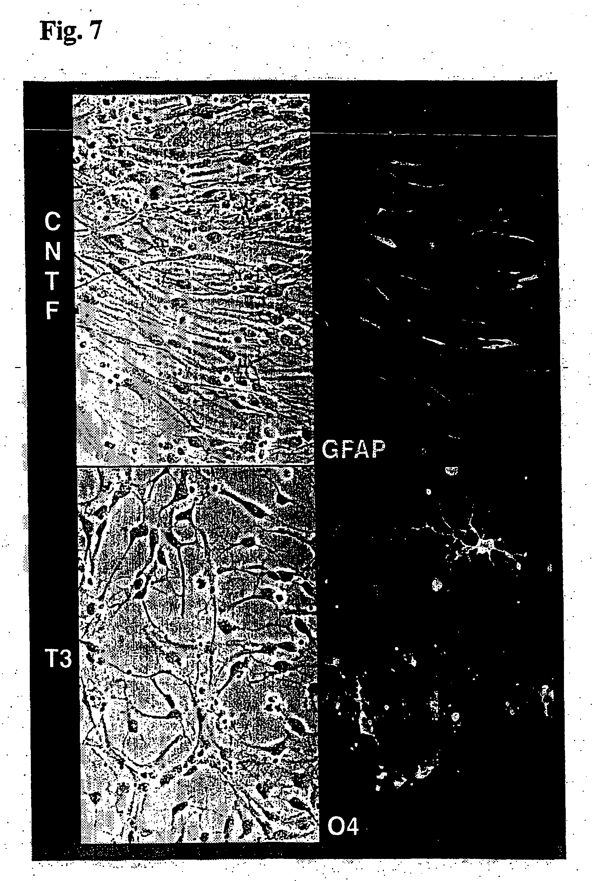

Abbildung 7 zeigt die Steuerung der glialen Differenzierung von aus ES Zellen gewonnenen glialen Vorläuferzellen durch Zugabe einzelner Faktoren. Hierbei wurde einer aus der ES Zellinie J1 gewonnenen sog. Touch-down Kultur, welche in Anwesenheit von EGF und FGF kultiviert worden war, zusätzlich die Faktoren CNTF (10 ng/ml) oder T3 (3 ng/ml) zugesetzt. 2 Tage später wurden die Wachstumsfaktoren entzogen, CNTF und T3 jedoch weiter täglich zugesetzt. Nach weiteren 5 Tagen wurden die Kulturen fixiert und mit Antikörpern gegen das astrozytäre Antigen GFAP und das oligodendrogliale Antigen 04 gefärbt. Die Abbildung, welche jeweils Immunfluoreszenz- und korrespondierende Phasenkontrast-Aufnahmen zeigt, verdeutlicht, daß die Zugabe von CNTF oder T3 zu den aus ES Zellen gewonnenen Vorläuferzellen die Differenzierung derselben beinflußt. In Anwesenheit von CNTF bildet der Großteil der Zellen eine astrozytäre Morphologie aus und exprimiert den astrozytären Marker GFAP. In Anwesenheit von T3 hingegen finden sich zahlreiche Zellen mit sternförmiger, oligodendroglialer Morphologie und Expression des oligodendroglialen Makers 04.

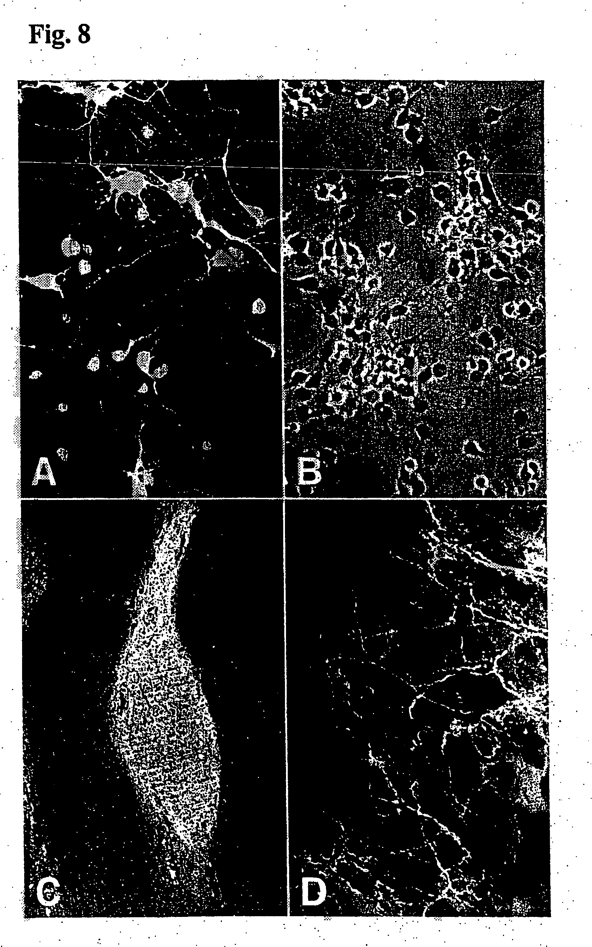

Abbildung 8 zeigt die neuronale Differenzierung von aus der ES Zellinie J1 gewonnenen neuralen Sphäroiden *in vitro* (A und B) und nach Transplantation in das Nervensystem einer Ibotensäure-behandelten Ratte (ein Tiermodell für die Huntington'sche Erkrankung, C und D). Hierbei wurden 5 Tage alte neurale Sphäroide in das Striatum einer adulten, Ibotensäure-behandelten Ratte implantiert. Ein Teil der Sphäroide wurde parallel *in vitro* durch Wachstumsfaktorentzug für 5 Tage differenziert und dann mit einem Antikörper gegen den Neurotransmitter GABA gefärbt (A, Immunfluoreszenz-Aufnahme; B, korrespondierende Phasenkontrast-Aufnahme). Es ist erkenntlich, daß zahlreiche der aus ES Zellen gewonnenen Nervenzellen den Neurotransmitter GABA exprimieren. 7 Wochen nach Implantation in das adulte Rattengehirn läßt sich ein vitales Transplantat mit neuronaler Differenzierung nachweisen (C und D). C zeigt eine Übersichtsaufnahme eines Transplantats, welches mit einem Antikörper gegen das Maus-spezifische neurale Antigen M6 gefärbt wurde (Immunfluoreszenz). Man erkennt ein homogen gefärbtes, in das Empfängergehirn inkorporiertes Transplantat. Bei diesem Tier war auch eine funktionelle Verbesserung aufgetreten. Das durch die Ibotensäure-Läsion hervorgerufene Rotationsverhalten konnte normalisiert werden. D zeigt von einem solchen Transplantat ausgehende, in das Empfängergehirn einsprossende Axone. Auch hierbei handelt es sich um eine Immunfluoreszenz-Untersuchung mit einem Antikörper gegen M6.

Als Ausgangsmaterial für die erfindungsgemäßen neuralen Vorläuferzellen können embryonale Stammzellen, z.B. der Maus, zunächst auf einem Rasen nicht teilungsfähiger embryonaler Fibroblasten in serumhaltigem Medium nach üblichen Verfahren (Hogan et al., Manipulating the Mouse Embryo, Cold Spring Harbor Press, New York, 1994) zu einer gewünschten Zellmenge proliferiert werden. Neben etablierten ES-Zellinien, z.B. die embryonalen Maus-Stammzellinien J1 (Li et al., Cell 69:915-926, 1992), R1 (Nagy et al. Proc. Natl. Acad. Sci. USA 90:8424-8428, 1993) und CJ7 (Swiatek & Gridley, Genes Dev. 7:2071-2084, 1993) können auch ES-Zellen aus Embryonen, beispielsweise aus 3 bis 4 Tage alten Blastozysten der Maus, gewonnen werden (Hogan et al., Manipulating the Mouse Embryo, Cold Spring Harbor Press, New York, 1994). Ferner können ES-Zellen auch aus anderen Spezies verwendet werden. Typische Beispiele isolierter ES-Zellen umfassen Ratte (lannaconne et al., Dev. Biol. 163:288-292, 1994), Hamster (Doetschman et al., Dev. Biol. 127:224-227, 1988), Vogel (Pain et al., Development 122:2339-2348, 1996), Fisch (Sun et al., Mol. Mar. Biol. Biotechno. 4:193-199, 1995), Schwein (Wheeler, Reprod. Fertil. Dev. 6:563-568, 1994), Rind (First et al., Reprod. Fertil. Dev. 6:553-562), Primaten (Thomson et al., Proc. Natl. Acad. Sci. USA 92:7844-7848, 1995) oder ES-Zellen aus menschlichem Embryonalgewebe. Mehrere Monate nach Einreichung der prioritätsbegründenden deutschen Patentanmeldung Nr. 197 56 864.5 gelang es zwei Forschergruppen, ES Zellen bzw. ES-Zell-ähnliche Stammzellen aus menschlichem Embryonalgewebe zu isolieren (Thomson et al., Science 282: 1145-1147, 1998; Shamblott et al., Proc. Natl. Acad. Sci. USA 95: 13726-13731, 1998).

Neuere Studien haben zudem gezeigt, daß Embryonen und damit auch embryonale Stammzellen durch Transplantation von Zellkernen aus Zellen eines ausgereiften Organismus in unbefruchtete Eizellen erhalten werden können (Wilmut et al., Nature 385:810-813, 1997). Für den Fachmann ist ersichtlich, daß eine Kombination der Kerntransplantation mit dem erfindungsgemäßen Verfahren die Herstellung autologer neuraler Vorläuferzellen aus differenzierten Zellen desselben Organismus erlaubt. Da die Erzeugung von Embryonen durch Transplantation von aus differenzierten Zellen gewonnenen Zellkernen in unbefruchtete Eizellen bereits an großen Säugetieren wie dem Schaf gezeigt wurde (Wilmut et al., Nature 385:810-813, 1997), ist dieses Verfahren auch bei menschlichen Zellen durchführbar. Ferner können ES-Zellen oder ES-Zell-ähnliche Zellen aus embryonalen Keimzellen *(embryonic germ cells)* erhalten werden. Die nach dem für diese Patentanmeldung geltenden Prioritätsdatum erschienenen Arbeiten über die Isolierung von ES-Zellen aus menschlichen Keimbläschen (Blastozysten; Thomson et al., Science 282: 1145-1147, 1998) bzw. von ES-Zell-ähnlichen Stammzellen aus humanen embryonalen Keimzellen (Shamblott et al., Proc. Natl. Acad. Sci. USA 95: 13726-13731, 1998) weisen darauf hin, daß diese Verfahren auf den Menschen übertragbar sind.

Von Feeder-Zellen abhängige ES-Zellen können dann z.B. nach dem von Okabe et al. beschriebenen Verfahren in Gelatine-beschichteten Schalen ausgesät und anschließend in unbeschichteten Petrischalen in der Abwesenheit von LIF zu Embryoid Bodies aggregiert werden (Okabe et al., Mech. Dev. 59:89-102, 1996). Drei bis vier Tage alte Embryoid Bodies können dann auf Zellkulturschalen ausplattiert und für 4-8 Tage in einem serumfreien Medium (ITSFn Medium) gezüchtet werden (Okabe et al., Mech. Dev. 59:89-102, 1996). Während dieser Zeit kommt es zu massivem Zelltod unter nicht neuralen Zellen.

Nach 4-8 Tagen in ITSFn Medium können die Zellen in eine Einzelzellsuspension trituriert (wiederholtes Aufziehen und Ablassen von Flüssigkeit mittels einer enghalsigen Pipette) und in N3FL Medium überführt werden. Es handelt sich hierbei um ein serumfreies Medium, welchem bFGF zugesetzt wird (Okabe et al., Mech. Dev. 59:89-102, 1996). bFGF hat einen proliferativen Effekt auf neurale Vorläuferzellen.

Nach 4-8 Tagen in N3FL Medium beginnt die eigentliche Bereitstellung glialer und neuronaler Vorläuferzellpopulationen in Form eines Zellrasens. Im Gegensatz zu neuronalen Zellen zeichnen sich Gliazellen und insbesondere ihre unmittelbaren Vorläufer durch eine starke Proliferationsfähigkeit aus. Das erfindungsgemäße Verfahren nützt diese Eigenschaft aus. Die vorstehend beschriebenen N3FL-Kulturen werden nacheinander in verschiedene serumfreie, Wachstumsfaktor-haltige Medien überführt. Während dieser Phase kommt es zu einer sehr starken Anreicherung bipotenter oligo-astrozytärer Vorläuferzellen bei gleichzeitiger Differenzierung und Elimination primitiver embryonaler und nicht-neural differenzierter Zellen.

Hierzu werden die in N3FL Medium kultivierten Zellen in üblicher Weise mechanisch von der Kulturschale gelöst, in eine Einzelzellsuspension trituriert und in serumfreies Medium überführt, welches z.B. die Wachstumsfaktoren bFGF und EGF (N3EFL Medium) enthält. In diesem Medium werden die Zellen bis zur Subkonfluenz als Zellrasen propagiert.

Die erhaltenen Zellen können nach etwa zwei Passagen in diesem Medium für Transplantationszwecke verwendet werden. Für eine weitergehende Aufreinigung kann jedoch ein zusätzlicher Schritt durchgeführt werden.

Die in N3EFL Medium kultivierten Zellen können auch erneut in üblicher Weise von der Kulturschale abgelöst, in eine Einzelzellsuspension trituriert, und erneut in einem serumfreien Medium ausplattiert werden, das eine zweite Wachstumsfaktorkombination z.B. bFGF und PDGF enthält.

Die so erhaltenen gereinigten neuralen Vorläuferzellen können weiteren Passagen in diesem Medium unterworfen werden. Nach etwa zwei Passagen besteht die Population aus gereinigten neuralen Vorläuferzellen, die direkt oder vorzugsweise nach Isolierung für z.B. remyelinisierende Transplantate verwendet werden können. Mikroskopisch erkennt man eine homogene Population bipolarer bis sternförmiger Zellen, welche die Kulturschale in Form eines gleichmäßig verteilten Zellrasens ausfüllen. Zu diesem, aber auch bereits zu einem früheren Zeitpunkt kann die neurale VorläuferzeUpopulation in serumfreiem Gefriermedium gefroren und zu einem späteren Zeitpunkt in üblicher Weise wieder aufgetaut werden, ohne ihre Vorläuferzelleigenschaften zu verlieren. Werden die Wachstumsfaktoren für mehrere Tage nicht zugesetzt, d.h. entzogen, kommt es zu einer beginnenden Differenzierung *in vitro.* Immunhistochemisch lassen sich dann neben Markerantigenen für neurale Vorläuferzellen (z.B. Nestin), auch oligodendrogliale (z.B. 04) und astrozytäre (z.B. GFAP) Antigene nachweisen. 04-positive Zellen können dabei bereits 30 - 70% und GFAP-positive Zellen 20 - 40% der Zellpopulation ausmachen. Darüber hinaus finden sich unter diesen Bedingungen Zellen, welche neuronale Antigene exprimieren.

Nach 4-8 Tagen in N3FL Medium beginnt ebenfalls die eigentliche Bereitstellung glialer und neuronaler Vorläuferzellpopulationen in Form von neuralen Sphäroiden.

Zur Anreicherung neuraler Zellen werden die in N3FL Medium kultivierten Zellen in üblicher Weise mechanisch von der Kulturschale gelöst, in eine Einzelzellsuspension trituriert und diese in unbeschichteten Zellkulturschalen in serumfreiem Medium kultiviert, welches z.B. die Wachstumsfaktoren bFGF und EGF enthält. Dabei entstehen innerhalb weniger Tage sphäroide Zellaggregate (neurale Sphäroide), welche zum überwiegenden Teil aus Nestin-positiven neuralen Vorläuferzellen bestehen. Die neuralen Sphäroide können freischwimmend im Zellkulturüberstand propagiert werden. Differenzierte neurale und nicht neurale Zellen zeigen hingegen eine starke Tendenz, sich an die Oberfläche der Zellkulturschalen anzuheften.

Sobald sich kleine Sphäroide gebildet haben, werden diese der Kultur entnommen und in neue unbeschichtete Zellkulturschalen überführt. Solche Sphäroide können bereits nach wenigen (5-7) Tagen für Transplantationszwecke verwendet werden. Hierbei differenzieren die in den Sphäroiden enthaltenen neuralen Vorläuferzellen in reife neurale Zellen aus, welche das Empfängergehirn innervieren. Undifferenzierte kleine Sphäroide können in serumfreiem Gefriermedium in flüssigem Stickstoff gefroren werden und zu einem späteren Zeitpunkt aufgetaut und differenziert werden.

Ein weiterer Gegenstand der Erfindung sind differenzierte neurale Zellen in Form von Sphäroiden, die sich zur Transplantation eignen. Sie können durch *in vitro*-Induktion der Differenzierung von erfindungsgemäßen Vorläuferzellen mit neuronalen oder glialen Eigenschaften hergestellt werden. Hierzu werden die Wachstumsfaktoren entzogen und die Sphäroide mit den undifferenzierten neuralen Vorläuferzellen in z.B. mit Polyornithin und Fibronektin beschichtete Zellkulturschalen ausgesät. Die Sphäroide heften sich dann rasch an die Oberfläche der Zellkulturschale an und bilden neben Nestin-positiven neuralen Vorläuferzellen Neurone, Astrozyten und Oligodendrozyten. Die anhaftenden Nervenzellen können weiter ausgereift werden und exprimieren dann eine Vielzahl neuronaler Marker, beispielsweise MAP2, β-III-Tubulin, Synapsin, Cholinacetyltransferase, Tyrosinhydroxylase, GABA, Glutamat, Serotonin, Peripherin und Calbindin. Die Ausreifung und das Überleben der ausgereiften Neurone kann durch Zugabe von Neurotrophinen, z.B. Brain-Derived Neurotrophic Factor (BDNF) oder Neurotrophin-3 (NT-3) zum Zellkulturmedium begünstigt werden.

Die Sphäroide können bis zu mehreren Wochen in serumfreiem Medium, welches z.B. die Wachstumsfaktoren bFGF und EGF enthält, in Suspensionskultur gehalten werden. Die Sphäroide können dabei soweit an Größe zunehmen, daß sie auch makroskopisch leicht zu erkennen sind. Innerhalb der Sphäroide kommt es unter solchen Bedingungen zu einer zunehmenden Zelldifferenzierung. Eine derartige Differenzierung von ES-Zellen in freischwimmenden Sphäroiden macht es physikalisch möglich, aus ES-Zellen gewonnene Neurone auch in differenzierterem Zustand zu transplantieren, z.B. in Form solcher Sphäroide. Dies ist nach Differenzierung von einer Kulturschale anhaftenden Neuronen nicht möglich, da die zahlreichen der Oberfläche anhaftenden Fortsätze beim Ablösen in der Regel beschädigt und die Neurone dadurch zerstört werden.

Während der letzten Jahre sind zahlreiche Faktoren identifiziert worden, die in der Lage sind, auf die Differenzierung von Nervenzellverbänden einzuwirken. Solche Faktoren können beispielsweise innerhalb von Nervengewebe eine Polarisierung herbeiführen. So wurde beispielsweise gezeigt, daß das Produkt des Gens *Sonic hedgehog* in Nervengewebe einen ventralen Phänotyp induzieren kann (Ericson et al., Cell 81 1:747-756, 1995). Es ist zu erwarten, daß derartige Faktoren auch an künstlich aus ES-Zellen hergestellten neuralen Zellverbänden wirksam sind. Für den Fachmann ist ersichtlich, daß die Applikation derartiger Phänotypbestimmender Faktoren es erlauben wird, Neurone und Glia von einem spezifischen Phänotyp gezielt herzustellen. Ein Beispiel könnte die gezielte Induktion von Nervenzellen von ventralem mesencephalem Phänotyp sein, wie sie für die Transplantation z.B. von Parkinson-Patienten benötigt werden. Eine derartige Induktion dopaminerger ventraler mesencephaler Neurone durch Sonic Hedghog wurde in Fragmenten natürlich gewachsenen Nervengewebes bereits nachgewiesen (Wang et al., Nature Med. 1:1184-1188, 1995).

Für die Herstellung glialer Vorläuferzellen aus von ES-Zellen erhaltenen neuralen Sphäroiden werden die Sphäroide solange in Wachstumsfaktor-haltigem serumfreiem Medium in Suspensionskultur gehalten, bis sie beginnen, sich leicht an die unbeschichtete Oberfläche der Zellkulturschale anzuheften. Dabei können als Wachstumsfaktoren z.B. bFGF und EGF (allein und in Kombination) verwendet werden. Nach Anheften der Sphäroide wandern Zellen von glialer Morphologie aus den Sphäroiden auf die Zellkulturplatten aus (sog. Touch-down Zellen). Die Entstehung dieser Zellpopulation ist ungeklärt. Es ist jedoch anzunehmen, daß im Laufe der zunehmenden Zelldifferenzierung innerhalb der Sphäroide gliale Vorläuferzellen gebildet werden, die sich durch ein stärkeres Adhesions- und Migrationsverhalten auszeichnen. Solche Touch-down Zellen-produzierende Sphäroide können als Generatoren für gliale Vorläuferzellen benützt werden. Hierzu werden die Sphäroide für kurze Zeit (< 1 Tag) in unbeschichteten Zellkulturschalen kultiviert. Sobald sich gliale Zellen abgesetzt haben, werden die Sphäroide wieder mechanisch von der Schale gelöst und in eine neue Schale überführt. Zurück bleibt in solchen Fällen ein Ring glialer Vorläuferzellen, der weiter in Anwesenheit von Wachstumsfaktoren, beispielsweise bFGF und EGF, allein oder in Kombination, vermehrt werden kann.

Derartig gewonnene "Touch-down Zellen" zeigen eine Expression des neuralen Antigens A2B5 (Eisenbarth et al., Proc. Natl. Acad. Sci. USA 76:4913-4917, 1979) und differenzieren nach Wachstumsfaktorentzug in Astrozyten und Oligodendrozyten aus. Immunhistochemisch lassen sich dann Markerantigene für Oligodendrozyten (z.B. 04) und Astrozyten (z.B. GFAP) nachweisen. Undifferenzierte "Touch-down Zellen" können in serumfreiem Gefriermedium in flüssigem Stickstoff gefroren werden, ohne ihr Proliferations- und Differenzierungsvermögen zu verlieren. Derartig gewonnene gliale Vorläuferzellen können auch für Transplantationen ins Nervensystem verwendet werden

Die Differenzierung der aus den ES-Zellen gewonnenen glialen Vorläuferzellen kann durch Zugabe einzelner Faktoren beeinflußt werden. Zugabe von CNTF (ciliary neurotrophic factor) kurz vor und während des Wachstumsfaktor-Entzugs führt zu einer überwiegend astrozytären Differenzierung. Zugabe des Schilddrüsenhormons T3 während dieser Phase führt zu einer vermehrten Differenzierung in Oligodendrozyten. Die Zugabe von serumhaltigem Medium während oder nach Wachstumsfaktor-Behandlung führt zu einem starken Anstieg der Zahl astrozytärer Zellen in diesen Kulturen.

Dem Fachmann ist ersichtlich, daß die gefrorenen und wieder aufgetauten neuralen Vorläuferzellen ebenfalls für Transplantate verwendet werden können. Zu einem früheren Zeitpunkt eingefrorenen neuralen Vorläuferzellpopulationen werden nach dem in üblicher Weise durchgeführten Auftauen den entsprechenden Passagen unterzogen, um die homogene Population bipolarer bis sternförmiger Zellen zu erhalten.

Das erfindungsgemäße Verfahren kann ferner mit bekannten Zelltrennungs- und Sortierverfahren kombiniert werden. Beispielsweise können neurale Subpopulationen zu bestimmten Zeitpunkten des erfindungsgemäßen Verfahrens durch Immunfluoreszenz-gestützte Zellsortierung (FACS), Immunopanning oder ähnliche Verfahren abgetrennt werden. Eine detaillierte Sortierung und Subklassifizierung kann es ermöglichen, auf die Bedürfnisse des jeweiligen Patienten abgestimmte (und unter Umständen genetisch modifizierte) neurale Ersatzzellen *in vitro* zu erzeugen. Da sowohl ES-Zellen als auch die aus ihnen gewonnenen erfindungsgemäßen neuralen Vorläuferzellen ohne Verlust ihrer Eigenschaften tiefgefroren und wieder aufgetaut werden können, ist ein Aufbau entsprechender Zellbanken z.B. autologer Zellbanken möglich.

Die mit dem erfindungsgemäßen Verfahren erhältlichen neuralen Vorläuferzellen z.B. neuronale, oligodendrogliale und astrozytäre Zellen können in einer Menge und Reinheit hergestellt werden, wie sie z.B. für die Transplantation und Reparatur von Defekten im Nervensystem erforderlich sind. Die mit dem erfindungsgemäßen Verfahren erhältlichen erfindungsgemäßen neuralen Vorläuferzellen enthalten z.B. nur geringe bis keine Mengen von z.B. primitiven embryonalen und nicht-neuralen Zellen. Die Reinheit der erfindungsgemäßen neuralen Vorläuferzellen liegt weit über der Reinheit von etwa 85% wie sie mit dem Verfahren von Okabe et al. (Mech. Dev. 59:89-102, 1996) erreicht wurde. Mit dem erfindungsgemäßen Verfahren können neurale Vorläuferzellen mit einer Reinheit von bis zu 100% erhalten werden. Ferner können mit dem erfindungsgemäßen Verfahren große Mengen neurale Vorläuferzellen gewonnen werden, ohne wie bisher auf Hirngewebe zurückgreifen zu müssen. Die erfindungsgemäßen neuralen Vorläuferzellen können aus ES-Zellen, z.B. der Maus, Ratte, Hamster, Vogel, Fisch, Schwein, Rind, Primaten oder Mensch erhalten werden. Die ES-Zellen können als ES-Zellinie vorliegen oder aus Embryonen gewonnen werden. Ferner können die ES-Zellen durch Proliferation von Oocyten gewonnen werden. Die Oocyten können z.B. entkernt und mit einem Zellkern aus z.B. ausdifferenziertem Gewebe injiziert werden, so daß autologe Oocyten zur Gewinnung von ES-Zellen verwendet werden. ES-Zellen bzw. ES-Zell-ähnliche Zellen können auch aus embryonalen Keimzellen *(embryonic germ cells)* erhalten werden. Die ES-Zellen können ferner in üblicher Weise gentechnisch modifiziert werden. Z.B. kann durch homologe Rekombination ein defektes Gen durch ein "normales" Gen ersetzt werden. Ferner können defekte Gene auf übliche Weise deletiert werden. Diese Verfahren sind z.B. in der Maus vielfach verwendet worden und sind Stand der Technik.

Da ES-Zellen sich rasch teilen und routinemäßig genetischen Modifikationen unterzogen werden, ist es auch möglich, große Mengen an genetisch modifizierten neuronalen und glialen Vorläuferzellen zu erhalten. In Verbindung mit dem ausgeprägten Wanderungsverhalten neuronaler und glialer Vorläuferzellen kann dies beispielsweise dafür benutzt werden, große Abschnitte des Nervensystems mit genetisch modifizierten Vorläuferzellen zu besiedeln, welche lokal fehlende Substanzen substituieren oder Polypeptide mit protektiver oder anderer Wirkung sezemieren. Die genetische Modifizierbarkeit der Zellen kann auch dazu benutzt werden, die Expression von Abstoßungsreaktionen hervorrufenden Oberflächenantigenen durch Elimination der entsprechenden Gene zu beseitigen, was einen breiten klinischen Einsatz von aus ES-Zellen abgeleiteten Vorläuferzellen ohne Immunsuppression erlaubt.

Die erfindungsgemäßen neuralen Vorläuferzellen können als medizinische Arbeitsmittel zur Therapie von neuralen Defekten verwendet werden. Ein typisches Beispiel zur Verwendung der erfindungsgemäßen neuralen Vorläuferzellen ist die Rekonstitution funktionell defizienter oder verlorengegangener Nervenzellen durch transplantierte neurale Vorläuferzellen.

Um beispielsweise verlorengegangene Neurone zu rekonstituieren und damit verbundene neurologische Defizite zu bessern, werden z.B. die erfindungsgemäßen Sphäroide nach z.B. 4-7 Tagen in Suspensionskultur in Gehirnregionen implantiert, welche einen Verlust von Nervenzellen aufweisen. In derartigen Transplantaten lassen sich z.B. 6 Wochen nach Operation ausgereifte Nervenzellen beobachten, welche das Empfängergehim innervieren. Die von den transplantierten Neuronen ausgehenden, in das Hirngewebe des Empfängers einsprossenden Axone können z.B. mit Antikörpern gegen Donor-spezifische neurale Antigene identifiziert werden. Diese Rekonstitution neuronaler Zellen ist funktionell. Dies läßt sich beispielsweise durch Verhaltenstests an Ibotensäure-behandelten Ratten vor und nach Transplantation belegen. Hierbei werden durch stereotaktische Injektion des Neurotoxins Ibotensäure in das Striatum zunächst große Mengen striataler Neurone zerstört. Der resultierende Defekt hat Ähnlichkeit zu der bei Menschen auftretenden Huntington'schen Erkrankung, weshalb dieses System auch vielfach als Tiermodell für die Huntington'sche Erkrankung benützt wird. Nach einseitiger Ibotensäureläsion läßt sich in den operierten Tieren aufgrund der eingetretenen Asymmetrie durch Injektion bestimmter Drogen, z.B. Amphetamin, ein Rotationsverhalten induzieren, welches auch quantifizierbar ist. Ein Nervenzell-reiches Transplantat ist imstande, das Rotationsverhalten zu normalisieren. Dabei scheint insbesondere die Zahl der im Transplantat vorhandenen GABAergen Nervenzellen von Bedeutung zu sein. Das Ibotensäure-Läsionsmodell der Ratte und die Erfassung der funktionellen Kapazität neuraler Transplantate über eine Analyse des Rotationsverhaltens der Tiere sind Stand der Technik (Björklund et al., aus: Functional Neural Transplantation, Seiten 157-195, Raven Press, New York, 1994). Werden die erfindungsgemäßen neuralen Sphäroide in das Gehirn Ibotensäure-behandelter Ratten implantiert, kommt es postoperativ zu einer deutlichen Besserung des abnormen, durch die Ibotensäure-Läsion verursachten Rotationsverhaltens der Empfänger. Transplantationen von z.B. neuralen Zellen ins menschliche Nervensystem sind heute bereits klinisch durchführbar (Olanow et al., TINS 19:102-109, 1996).

Die erfindungsgemäßen, entweder aus den neuralen Sphäroiden oder aus dem Zellrasen hergestellten glialen Vorläuferzellen können ebenfalls als medizinische Arbeitsmittel zur Therapie von neuralen Defekten verwendet werden. Ein typisches Beispiel zur Verwendung der erfindungsgemäßen glialen Vorläuferzellen ist die Remyelinisierung demyelinisierter Hirnabschnitte durch transplantierte neurale Vorläuferzellen. Da demyelinisierende Erkrankungen oftmals große Abschnitte des Zentralen Nervensystems (ZNS) miteinbeziehen, können die erfindungsgemäßen glialen Vorläuferzellen, welche sich durch ein ausgeprägtes Wanderungsverhalten auszeichnen, ins ZNS transplantiert werden. Dabei kann eine lokalisierte Injektion genügen, um große Abschnitte des ZNS mit diesen Zellen zu infiltrieren und eine großflächige Remyelinisierung zu erreichen. Ein typisches Beispiel einer möglicherweise derartig therapierbaren Erkrankung ist die multiple Sklerose (MS). Bei dieser Erkrankung, deren Pathogenese noch ungeklärt ist, kommt es typischerweise zu einer multifokalen Demyelinisierung verschiedenster Hirnabschnitte. Durch die Transplantation der erfindungsgemäßen neuralen Vorläuferzellen kann eine Remyelinisierung solcher Defekte bewirkt werden. Das ausgeprägte Wanderungsverhalten der erfindungsgemäßen neuralen Vorläuferzellen kann dabei derartig ausgenutzt werden, daß von einer oder wenigen Implantationsstellen aus zahlreiche demyelinisierte Areale erreicht und repariert werden können.

Um beispielsweise eine Myelinisierung myelin-defizienter Hirnabschnitte herbeizuführen, werden die noch unter Wachstumsfaktor-Behandlung stehenden aus Monolayern (Zellrasen) oder neuralen Sphäroiden gewonnenen erfindungsgemäßen Zellen z.B. trypsinfrei von den Kulturschalen abgelöst und zu einer Einzelzellsuspension trituriert. Werden diese Zellsuspensionen in myelindefiziente Gehirnregionen implantiert, lassen sich beispielsweise etwa drei Wochen nach Implantation von den implantierten Zellen abstammende Oligodendrozyten und Astrozyten nachweisen. Die Myelinisierung der Nervenzellfortsätze des Empfängers läßt sich durch eine von den Donorzellen ausgehende Einscheidung dieser Fortsätze nachweisen, wobei in diesen Einscheidungen für Myelin charakteristische Proteine immunhistochemisch nachgewiesen werden können z.B. basisches Myelinprotein (MBP) und Proteolipid Protein (PLP).

Die erfindungsgemäßen neuralen Vorläuferzellen können ferner für die *in vitro* Produktion von klinisch und gewerblich nutzbaren Faktoren z.B. Polypeptiden benützt werden.

Die folgenden Beispiele erläutern die Erfindung und sind nicht als einschränkend zu verstehen.

### Beispiel 1:

### 1.1. ES-Zell Proliferation

J1-ES-Zellen (Li et al., Cell 69:915-926, 1992) wurden zunächst auf einem Rasen von mitotisch inaktivierten embryonalen Fibroblasten in einem DMEM Medium (Life Technologies Nr. 11965) mit 20% fötalem Kälberserum (FB S, Life Technologies Nr. 10119) in Anwesenheit von 1.000 U/ml LIF (Life Technologies Nr. 13275) nach Standardmethoden (Hogan et al., Manipulating the Mouse Embryo, Cold Spring Harbor Press, New York, 1994) proliferiert. Neben FBS und LIF enthielt dieses Medium Standardkonzentrationen (Okabe et al., Mech. Dev. 59:89-102, 1996) nicht essentieller Aminosäuren (Life Technologies Nr. 11140) und der Nukleoside Adenosin (Sigma Nr. A-4036), Guanosin (Sigma Nr. G-6264), Cytidin (Sigma Nr. C-4654), Uridin (Sigma Nr. U-3003) und Thymidin (Sigma Nr. T-1895) sowie 0.1 mM 2-Mercaptoethanol (Sigma Nr. M-7522), 10 mM L-Glutamin (Sigma Nr. G-5763) und 25 mM HEPES (Sigma Nr. H-0763). In diesem wie in allen folgenden Schritten wurden die Zellen bei 37°C unter 5% CO₂ bei gesättigter Luftfeuchtigkeit (>85%) kultiviert.

### 1.2. Entfernung der Feeder Zellen

Sobald die ES-Zellen subkonfluent geworden waren, wurde die Kultur einmal mit 0,04% EDTA in PBS-Puffer gespült und die Zellen durch Zugabe einer Lösung von 0,05% Trypsin (Life Technologies Nr. 35400) und 0,04% EDTA in PBS abgelöst. Die Zellen wurden dann mehrfach durch eine Pasteurpipette zu einer Einzelzellsuspension trituriert und das Trypsin durch Zugabe serumhaltigen Mediums neutralisiert. Hierbei wurde der Trypsinlösung eine äquivalente Menge des bislang verwendeten Mediums zugesetzt. Nach Zentrifugation (5 min, 300 × g, RT) wurden die Zellen in dem bisher verwendeten Medium auf mit 0,2% Gelatine (Sigma Nr. G-2500) beschichtete 6 cm Zellkulturschalen ausplatiert (ca. 6 × 10⁶ Zellen pro 6 cm Schale) und unter Anwesenheit von LIF (1.000 U / ml) weiter gezüchtet.

### 1.3. Herstellung von Embryoid Bodies

Sobald die Zellen erneut subkonfluent geworden waren (etwa nach 2-3 Tagen), wurden sie durch Zugabe einer Lösung von 0,05% Trypsin und 0,04% EDTA in PBS (ca. 1,5 ml pro 6 cm Schale) von der Gelatine abgelöst. Nach Ablösen der ES-Zell-Kolonien wurde das Trypsin durch Zugabe von DMEM-Medium mit 10% FBS neutralisiert (ca. 6,5 ml pro 6 cm Schale; dieses Medium entspricht dem oben beschriebenen Medium, enthält aber nur 10% FBS und kein LIF). Diese Suspension wurde dann auf 8 unbeschichtete Bakterienkultur-Petrischalen (Nunc Nr. 240045) verteilt und in einem Gesamtvolumen von ca. 4 ml DMEM/10% FBS pro 6 cm Schale weiter kultiviert.

### 1.4. Platieren der Embryoid Bodies

Nach 3-4 Tagen wurden die sich gebildeten Embryoid Bodies durch einfache (1 × g) Sedimentation für 5 min in einem 50 ml Zellkulturröhrchen gesammelt. Der Überstand wurde verworfen und die Embryoid Bodies derart auf 10 cm Zellkulturschalen verteilt, daß nach Absenken der Embryoid Bodies etwa 50% der Fläche der Zellkulturschale bedeckt waren (Embryoid Bodies aus ca. 4-6 Bakterienkulturschalen reichten in der Regel für die Beschickung einer 10 cm Zellkulturschale aus). Die Embryoid Bodies wurden über Nacht in DMEM/10% FBS (10 ml pro 10 cm Schale) kultiviert.

### 1. 5. Transfer in ITSFn Medium

Am folgenden Tag wurden die mittlerweile adhärenten Embryoid Bodies 3 x in DMEM/F12 (Dulbecco's Modified Eagle Medium / Nutritient Mix F 12 (1:1; Life Technologies, Nr. 11320) Medium gewaschen. Anschließend wurden 10 ml ITSFn Medium pro 10 cm Schale zugegeben. ITSFn Medium besteht aus DMEM/F 12, dem 5 µg/ml Insulin (Intergen Nr. 540101), 50 µg/ml Apo-Transferrin (Intergen Nr. 445275), 30 nM Seleniumchlorid (Sigma Nr. S-5261), 5 µg/ml Fibronektin (Life Technologies Nr. 33010) und Penicillin/Streptomycin (100 IU/ml 100 µg/ml; Life Technologies Nr. 15140) zugesetzt wurden. Die Zellen wurden 4 - 7 Tage in diesem Medium kultiviert, wobei das Medium jeden zweiten Tag ersetzt wurde. Während der Kultivierung in ITSFn Medium kommt es zu massivem Zelltod unter den nicht-neuralen Zellen und zur Differenzierung in kleinen Grüppchen liegender neuraler Vorläuferzellen. Die hier beschriebene Etablierung von ITSFn Kulturen ist Stand der Technik (Okabe et al., Mech. Dev. 59:89-102, 1996).

### 1.6. Transfer in N3FL Medium

Die in ITSFn kultivierten Zellen wurden mittels einer Lösung von 0,05% Trypsin und 0,04% EDTA in PBS von der Kulturschale abgelöst und das Trypsin durch Zugabe einer äquivalenten Menge serumhaltigen Mediums (DMEM/10% FBS) neutralisiert. Nach Sedimentation (5 min, 300 × g, RT) wurden die Zellen in Calcium- und Magnesium-freier Hanks Buffered Salt Solution (CMF-HBSS, Life Technologies Nr. 14180) mit 0,1% DNase (Worthington Nr. 2139) resuspendiert (ca. 3 ml auf ein aus einer 10 cm Schale gewonnenes Pellet) und mit Hilfe von Pasteurpipetten zu einer Einzelzellsuspension trituriert. Zu diesem Zweck wurden mehrere Pasteurpipetten verwendet, deren Mündungen mit Hilfe einer Flamme abgerundet und verkleinert worden waren (auf 0,8 mm, 0,5 mm und 0,2 mm). Dabei wurde die Zellsuspension mehrere Male durch Pasteurpipetten mit abnehmender Mündungsgröße zu einer homogenmilchigen Suspension trituriert. Etwaige in der Suspension verbliebene Zellaggregate wurden vor Weiterbehandlung der Suspension durch Sedimentation (1 × g, 5 min) am Boden des Gefäßes gesammelt und verworfen. Die Zellen wurden dann zentrifugiert (300 × g, 5 min, RT) und in einer Dichte von ca. 30.000 Zellen/cm² auf zuvor mit Polyornithin beschichtete Zellkulturschalen in folgendem Medium ausgesät: DMEM/F12 (1:1; Life Technologies Nr. 11320), 25 µg/ml Insulin, 50 µg/ml humanes Apo-Transferrin, 20 nM Progesteron (Sigma Nr. P-8783), 100 µM Putrescin (Sigma Nr. P-5780), 30 nM Seleniumchlorid, 1 µg/ml Laminin (Life Technologies Nr. 23017), Penicillin/Streptomycin (100 IU/ml / 100 µg/ml), 10 ng/ml humanes rekombinantes bFGF (R&D Systems Nr. 233-FB). Dieses Medium entspricht dem von Okabe et al. geschilderten N3FL Medium (Okabe et al., Mech. Dev. 59:89-102, 1996). Für eine Polyornithin-Beschichtung wurden Zellkulturschalen mit einer Lösung von 15 µg/ml Polyornithin (Sigma Nr. P-3655) in H₂O für mindestens 2 Stunden gefüllt. Anschließend wurde die Polyornithin-Lösung abgesaugt und die Kulturschale dreimal mit PBS gespült. bFGF wurde täglich zu einer Endkonzentration von 10 ng/ml zugesetzt und das Medium jeden zweiten Tag erneuert.

### Beispiel 2: Gewinnung neuraler Vorläuferzellen

### 2.1. Transfer in N3EFL Medium

Nach 4-5 Tagen in diesem Medium wurden die Zellen unter Trypsin-freien Bedingungen mechanisch von der Kulturschale gelöst. Hierzu wurde die Kultur dreimal mit CMF-HBSS gespült, wobei der letzten Spülung 0,1% DNase zugesetzt wurden. Die Zellen wurden dann mit einem Zell-Lifter (Costar Nr. 3008) von der Platte abgeschabt und nach dem oben beschriebenen Modus mit Hilfe von Flammen-polierten Pasteur Pipetten zu einer Einzelzellsuspension trituriert. Nach Zentrifugation (300 × g, 5 min, RT) wurde der Ertrag einer 10 cm Zellkulturschale in ca. 5 zuvor mit Polyornithin beschichtete Zellkulturschalen in N3EFL Medium ausplatiert. Dieses setzte sich wie folgt zusammen: DMEM/F12 (1:1), 25 µg/ml Insulin, 100 µg/ml Transferrin, 20 nM Progesteron, 100 µM Putrescin, 30 nM Seleniumchlorid, 1 µg/ml Laminin, Penicillin/Streptomycin (100 IU/ml / 100 µg/mi), 10 ng/ml humanes rekombinantes bFGF und 20 nglml humanes rekombinantes EGF (R&D Systems Nr. 236-EG). bFGF und EGF wurden täglich zu einer Endkonzentration von 10 ng/ml für bFGF und 20 ng/mi für EGF zugesetzt. Das Medium wurde jeden zweiten Tag ersetzt, wobei Laminin nach dem zweiten Mediumwechsel nicht mehr zugesetzt wurde.

### 2.2. Transfer in N2FP Medium

Sobald die Zellen zu 90% konfluent geworden waren (nach 1-2 Wochen), wurden sie erneut unter Trypsin-freien Bedingungen mit einem Zell-Lifter mechanisch von der Kulturschale gelöst und mit Hilfe Flammen-polierter Pasteur Pipetten zu einer Einzelzellsuspension trituriert. Nach Zentrifugation (300 × g, 5 min, RT) wurde der Ertrag einer 10 cm Zellkulturschale in ca. 5 zuvor mit Polyornithin beschichtete Zellkulturschalen in N2FP Medium ausplatiert. Dieses setzte sich wie folgt zusammen: DMEM/F12 (1:1), 25 µg/ml Insulin, 100 µg/ml Transferrin, 20 nM Progesteron, 100 µM Putrescin, 30 nM Seleniumchlorid, Penicillin/Streptomycin (100 IU/ml / 100 µg/ml), 10 ng/ml humanes rekombinantes bFGF und 10 ng/ml rekombinantes humanes PDGF-AA (R&D Systems Nr. 221-AA). bFGF und PDGF-AA wurden täglich zugesetzt und das Medium jeden zweiten Tag erneuert.

### 2.3. Passagieren in N2FP Medium

Sobald die Zellen subkonfluent geworden waren, wurden sie trypsinfrei nach dem oben beschriebenen Modus 1:5 passagiert. Eine Triturierung der Zellen in DNase-haltiger Pufferlösung war hier meist entbehrlich. Nach mindestens zwei Passagen in diesem bFGF und PDGF-haltigen Medium bestand die Population aus neuralen Vorläuferzellen wie sie für remyelinisierende Transplantate verwendet werden konnten. Alternativ konnten die Zellen in diesem Stadium trypsinfrei von der Kulturschale gelöst und in serumfreiem Gefriermedium (Sigma Nr. C-6295) für eine spätere Verwendung tiefgefroren werden. Die gereinigten neuralen Vorläuferzellen in dem Medium können isoliert und in ein Injektionsmedium z.B. in Calcium- und Magnesium-freier Hanks Buffered Salt Solution (CMF-HBSS, Life Technologies Nr. 14180) aufgenommen werden.

### 2.4. In vitro Differenzierung der neuralen Vorläuferzellen

Für die *in vitro* Differenzierung der neuralen Vorläuferzellen wurden diese nach trypsinfreiem Passagieren in mit Polyornithin beschichtete Zellkulturschalen zu 50%iger Konfluenz ausplattiert und in dem unter Beispiel 2.2 genannten Medium, jedoch ohne bFGF und PDGF, für weitere 4-7 Tage kultiviert, wobei das Medium jeden zweiten Tag erneuert wurde. Die Kulturen wurden dann in 4% Paraformaldehyd-Lösung (Sigma Nr. P-6148) fixiert und einer Immunfluoreszenz-Untersuchung mit Antikörpern gegen das oligodendrogliale Antigen 04 (Boehringer Nr. 1518925, Verdünnung 1:10) und das astrozytäre Antigen GFAP (Chemicon, Nr. AB 1980, Verdünnung 1:100) unterzogen. Hierbei zeigten bereits 4 Tage nach Wachstumsfaktorentzug bis zu 32% der Zellen eine typisch oligodendrogliale Morphologie mit Expression von 04; bis zu 49% der Zellen exprimierten zu diesem Zeitpunkt GFAP. Darüber hinaus fanden sich Zellen, welche das neuronale Antigen β-III-Tubulin exprimierten (Antikörper von BAbCO, Nr. MMS-435P-250, Verdünnung 1:500).

### Beispiel 3: Gewinnung neuraler Sphäroide und daraus etablierter neuraler Vorläuferzellen

### 3.1. Etablierung neuraler Sphäroide in N2EF Medium

Nach 4-5 Tagen in dem in Beispiel 1.6 genannten N3Fl Medium wurden die Zellen unter Trypsin-freien Bedingungen mechanisch von der Kulturschale gelöst. Hierzu wurde die Kultur dreimal mit CMF-HBSS gespült, wobei der letzten Spülung 0,1% DNase zugesetzt wurden. Die Zellen wurden dann mit einem Zell-Lifter (Costar Nr. 3008) von der Platte abgeschabt und nach dem oben beschriebenen Modus mit Hilfe von Flammen-polierten Pasteur Pipetten zu einer Einzeizellsuspension trituriert. Nach Zentrifugation (300 x g, 5 min, RT) wurden die Zellen in einer Dichte von 1.200 - 12.000 Zellen/cm² in unbeschichteten Zellkulturschalen in folgendem Medium ausgesät: DMEM/F12 (1:1), 25 µg/ml Insulin, 100 µg/ml Transferrin, 20 nM Progesteron, 100 µM Putrescin, 30 nM Seleniumchlorid, Penicillin/Streptomycin (100 IU/ml/100 µg/ml), 20 ng/ml humanes rekombinantes bFGF und 20 ng/ml humanes rekombinantes EGF (R&D Systems Nr. 236-EG). bFGF und EGF wurden täglich zu einer Endkonzentration von 20 ng/ml zugesetzt und das Medium (sog. N2EF Medium) alle zwei Tage erneuert. Hierzu wurde das verbrauchte Medium abgezogen und bei 150 x g für 3 min bei RT zentrifugiert, um etwaige im Medium schwimmende Sphäroide zu sedimentieren. Das Pellet wurde in frischem Medium resuspendiert und wieder ausgesät. Nach dem zweiten Mediumwechsel wurden die Zellen in frischen unbeschichteten Zellkulturschalen ausgesät.

### 3.2. In vitro Differenzierung der gewonnenen neuralen Sphäroide

Fünf Tage alte, gemäß Beispiel 3.1 erhaltene neurale Sphäroide wurden bei 150 x g für 3 min bei RT zentrifugiert und in dem in Beispiel 3.1 genannten Medium, jedoch ohne bFGF und EGF, in mit Polyornithin und Fibronektin beschichteten Zellkulturschalen ausgesät. Für eine Doppelbeschichtung von Zellkulturschalen mit Polyornithin und Fibronektin wurden die Platten zunächst in der unter Beispiel 1.6 beschriebenen Weise mit Polyornithin beschichtet und dreimal mit PBS gespült. Dann wurden die Schalen mit einer Lösung von 1 µg Fibronektin / ml PBS beschickt und für 2-12 Stunden bei RT inkubiert. Die Fibronektin-Lösung wurde abgezogen und die Zellsuspension in die derartig beschichtete Schale ausgesät. Einen Tag nach Aussaat hatten sich die neuralen Sphäroide an die Oberfläche der Zellkulturschale angeheftet. Zu diesem Zeitpunkt wurde das Medium abgezogen, die Schale dreimal mit frischem Medium oder CMF-HBSS gewaschen, und neues Medium eingefüllt, welches im weiteren alle zwei Tage ersetzt wurde. Derartig differenzierte neurale Sphäroid-Kulturen wurden 5 Tage nach Plattieren fixiert und einer Immunfluoreszenzanalyse unterzogen. Hierbei verteilte sich die Expression neuraler Antigene wie folgt (Mittelwert der Zahl gefärbter Zellen ± SEM): Nestin-positive neurale Vorläuferzellen 66±3% (Antikörper von M. Marvin und R.D.G. McKay, NIH, Behtesda, USA, Verdünnung 1:1.000), β-III-Tubutin-positive Neurone 34±3% (Antikörper von BAbCO, Nr. MMS-435P-250, Verdünnung 1:500), GFAP-positive Astrozyten 30±2% (Antikörper von Chemicon, Nr. AB1980, Verdünnung 1:100), O4-positive Zellen mit oligodendroglialer Morphologie 6.2±1.7% (Antikörper von Boehringer, Nr. 1518925, Verdünnung 1:10), keines dieser Antigene aufweisende Zellen 2±0.7%. Weiter ließen sich in diesen für 5 Tage differenzierten Sphäroid-Kulturen in den entstandenen Nervenzellen Microtubule-Associated Protein 2 (MAP-2, Antikörper von Sigma, Nrs. M 4403 und M 1406, Verdünnung 1:200) und die Neurotransmitter GABA (Antikörper von Sigma, Nr. A-2052, Verdünnung 1:700) und Glutamat (Antikörper von Sigma, Nr. G-6642; Verdünnung 1:700) nachweisen. Der Anteil der GABA-positiven Neurone lag in manchen Präparationen bei bis zu 60%.

Wurden die Sphäroid-Kulturen über einen Zeitraum von 10 Tagen und mehr in Abwesenheit von Wachstumsfaktoren differenziert, liessen sich in den aus den Sphäroiden entstandenen Neuronen in Immunfluoreszenz-Untersuchungen zusätzlich Tyrosinhydroxylase (Antikörper von Eugene, Nr. TE101, Verdünnung 1:200), Cholinacetyltransferase (Antikörper von Chemicon, Nr. MAB305, Verdünnung 1:250), Serotonin (Antikörper von Eugene, Nr. NT102, Verdünnung 1:200), Synapsin (Antikörper von Dr. M. B. Kennedy, Pasadena, CA, USA, Verdünnung 1: 1.000), Peripherin (Antikörper von Chemicon, Nr. AB 1530, Verdünnung 1:1.000) und Calbindin (Antikörper von Sigma, Nr. C-8666, Verdünnung 1:100) nachweisen. Hierbei liessen sich Differenzierung und Überleben der Nervenzellen durch Zugabe der Neurotrophine Brain-Derived Neurotrophic Factor (BDNF; 20 ng/ml; Pepro Tech Inc. Nr. 450-02) und/oder Neurotrophin 3 (NT-3; Pepro Tech Inc. Nr. 450-03) steigern. In solchen über einen Zeitraum von 10 Tagen und mehr differenzierten Sphäroid-Kulturen kam es auch zu einer weiteren Ausreifung der Oligodendrozyten, in welchen sich dann in Immunfluoreszenz-Untersuchungen *cyclic nucleotide 3'-phosphodiesterase* (CNPase; Antikörper von Sigma, Nr. C-5922, Verdünnung 1:200) nachweisen liess.

### 3.3. Gewinnungg,lialer Vorläuferzellen aus neuralen Sphäroiden (sog. Touch-Down Kulturen)

Zur Gewinnung glialer Vorläuferzellen wurden die gemäß Beispiel 3.1 gewonnenen Sphäroide solange in dem unter Beispiel 3.1 genannten Wachstumfaktor-haltigen Medium kultiviert, bis sie begannen, spontan an die unbeschichtete Oberfläche der Zellkulturschalen zu adhärieren und Zellen aus den Sphäroiden auf die Oberfläche der Zellkulturschale ausgewandert waren. Dies war in der Regel nach einem Zeitraum von 10-14 Tagen der Fall. Sobald ein "Ring" ausgewanderter Zellen sichtbar wurde, wurden die der Zellkulturschale nur sehr locker anhaftenden Sphäroide durch Schütteln der Schale losgelöst und mittels einer Pipette aus der Kultur entfernt. Die so entfernten Sphäroide konnten in weitere unbeschichtete Zellkulturschalen transferiert und im selben Medium weiter gezüchtet werden, wobei sie erneut "Ringe" auswachsender glialer Vorläuferzellen erzeugten. Die in den unbeschichteten Zellkulturschalen zurückbleibenden glialen Vorläuferzellen wurden in dem Wachstumsfaktor-haltigen Medium weiter proliferiert und nach Erreichen einer etwa 80%igen Konfluenz trypsinfrei nach dem oben beschriebenen Modus 1:5 passagiert. So gewonnene gliale Vorläuferzellen zeigten in Immunfluoreszenz-Untersuchungen eine deutliche Expression des neuralen Antigens A2B5 (Antikörper von Boehringer, Nr. 1300 016, Verdünnung 1:200). Sie konnten in serumfreiem Gefriermedium (Sigma Nr. C-6295) in flüssigem Stickstoff kryokonserviert und zu einem späteren Zeitpunkt weiter proliferiert, transplantiert oder differenziert werden.

### 3.4. Steuerung der glialen Differenzierung durch Zugabe von Faktoren

Die Steuerung der glialen Differenzierung durch Zugabe einzelner Faktoren wurde wie folgt überprüft. Gemäß Beispiel 3.3 erhaltene, in der Anwesenheit von bFGF (20 ng/ml) und EGF (20 ng/ml) kultivierte gliale Vorläuferzellen wurden trypsinfrei passagiert und in mit Polyornithin beschichtete Zellkulturschalen ausgesät. Nach Erreichen einer etwa 50%igen Konfluenz wurden die Zellen für zwei Tage wie folgt behandelt.
1. Weiterkultivieren in EGF und bFGF-haltigem Medium
2. Weiterkultivieren in EGF und bFGF-haltigem Medium
3. Weiterkultivieren in EGF und bFGF-haltigem Medium unter zusätzlicher täglicher Zugabe von CNTF (Regeneron, Inc.; verwendete Endkonzentration 10 ng/ml)
4. Weiterkultivieren in EGF und bFGF-haltigem Medium unter zusätzlicher täglicher Zugabe von T3 (Sigma Nr. T 6397; verwendete Endkonzentration 3 ng/ml)

Nach 2 Tagen wurden in den Gruppen 2, 3 und 4 bFGF und EGF entzogen und die Zellen für weitere 5-7 Tage kultiviert, wobei das Medium etwa alle 2 Tage ersetzt wurde. Dann wurden alle Kulturen in 4% Paraformaldehyd-Lösung (Sigma Nr. P-6148) fixiert und einer Immunfluoreszenz-Untersuchung mit Antikörpern gegen das oligodendrogliale Antigen 04 und das astrozytäre Antigen GFAP unterzogen. Dabei ergaben sich folgende Mittelwerte (± SEM):

| | | |
|---|---|---|
| 1. | 04-positive Zellen mit oligodendroglialer Morphologie: GFAP-positive Zellen | < 1% |
| | | < 1% |
| 2. | O4-positive Zellen mit oligodendroglialer Morphologie: GFAP-positive Zellen | 8.2 ± 2.6% |
| | | 28 ± 6% |
| 3. | 04-positive Zellen mit oligodendroglialer Morphologie: GFAP-positive Zellen | 2.6 ± 1.8% |
| | | 78 ± 2.5% |
| 4. | 04-positive Zellen mit oligodendroglialer Morphologie: GFAP-positive Zellen | 17 ± 3.6% |
| | | 39 ± 5.7% |

Diese Daten zeigen, daß die Differenzierung der aus ES Zellen erhaltenen glialen Vorläuferzellen mit CNTF in eine astrozytäre und mit T3 in eine oligodendrogliale Richtung gelenkt werden kann.

### Beispiel 4: Transplantation oligodendroglialer/astrozytärer Vorläuferzellen

### 4.1. Herstellung einer Zellsuspension für die Transplantation

Die remyelinisierende Potenz dieser Zellen wurde durch folgendes Transplantationsexperiment überprüft: Subkonfluente Kulturen aus Beispiel 2.3, welche noch bis zum Vortag der Transplantation bFGF und PDGF erhalten hatten, wurden trypsinfrei (mit einem Zell-Lifter) von der Kulturschale gelöst, bei 300 × g über 5 min sedimentiert und in CMF-HBSS mit 0,1% DNase resuspendiert. Die Zellen wurden dann mit Flammen-polierten Pasteurpipetten zu einer Einzelzellsuspension trituriert, in einer Zählkammer gezählt und erneut sedimentiert. Sie wurden in einer Konzentration von ca. 50.000-100.000 Zellen/µl in CMF-HBSS, welche 2 g/L Glucose (Sigma Nr. G-7021) enthielt, resuspendiert und bis zur Transplantation (bis zu 4-6 Stunden) auf Eis gelagert.

### 4.2. Intraventrikuläre Transplantation ins embryonale Rattengehirn

Als Transplantatempfänger wurden embryonale myelindefiziente Sprague-Dawley Ratten (Ian Duncan. Department of Medical Sciences, School of Veterinary Medicine, University of Wisconsin-Madison, 2015 Linden Drive West, Madison, Wisconsin 53706, USA) verwendet.

Die frühe Transplantation während der Embryonalzeit hat den Vorteil, daß es trotz des Xenotransplantats nicht zu einer Abstoßungsreaktion kommt. Die myelindefizienten Empfängertiere haben den Vorteil, daß von den Donorzellen gebildetes Myelin einfach nachweisbar ist. Die Xenotransplantation selbst hat den Vorteil, daß die transplantierten Zellen einfach und mit hoher Sicherheit mit Hilfe speziesspezifischer DNA Proben nachgewiesen werden können (Brüstle et al., Neuron 15:1275-1285, 1995). Die Technik der intrauterinen Transplantation ins embryonale Gehirn ist Stand der Technik (Brüstle et al., Neuron 15:1275-1285, 1995; Brüstle et al., aus: Current Protocols in Neuroscience, John Wiley, New York, 1997). Zur Transplantation wurden schwangere Ratten am Embryonaltag 16 oder 17 mit einer intraperitonealen Injektion von Ketamin® (80 mg/kg) und Xylazin® (10 mg/kg) betäubt. Das Abdomen wurde eröffnet, und die einzelnen Embryonen unter Transillumination mit einer Faseroptik-Lichtquelle dargestellt. Wie beschrieben (vgl. Brüstle et al., Current Protocols in Neuroscience, John Wiley, New York, 1997) wurde die Zellsuspension in eine feine Glaskapillare mit einem Mündungsdurchmesser von 50-100 µm aufgezogen und die Glaskapillare durch den Uterus und die Schädeldecke in den Seitenventrikel des Embryos eingeführt. 2-9 µl der Zellsuspension (entsprechend 100.000-900.000 Zellen) wurden dann in das Ventrikelsystem injiziert. Die Glaskapillare wurde entfernt und die Bauchdecken nach Injektion mehrerer oder aller Embryonen wieder chirurgisch verschlossen. Derartig operierte Tiere werden spontan geboren. Da es sich um eine X-chromosomal rezessiv vererbte Erkrankung handelt, sind durchschnittlich 50% der männlichen Nachkommen myelindefizient. Diese Tiere beginnen in der dritten Lebenswoche stark zu zittern und sterben in der Regel in der vierten Lebenswoche.

### 4.3. Histologische Analyse der Transplantatempfäneer

Zum Nachweis der Myelinisierung wurden die Tiere in üblicher Weise in der dritten bis vierten Lebenswoche betäubt und durch eine transkardiale Perfusion mit einer 4%igen Paraformaldehydlösung (Sigma Nr. P-6148) in PBS in üblicher Weise fixiert. Die Gehirne wurden in üblicher Weise präpariert und über Nacht in der Fixationslösung bei 4°C nachfixiert. Im Anschluß daran wurden auf einem Vibratom 50 µm dicke Schnitte angefertigt. Die Donorzellen wurden mittels DNA *in situ* Hybridisierung mit einem DNA-Sondenmolekül für Maus Satelliten DNA nachgewiesen (Brüstle et al., Neuron 15:1275-1285, 1995). Von den Donorzellen gebildetes Myelin wurde über eine immunhistochemische Detektion von Myelinproteinen in Fortsätzen hybridisierter Zellen nachgewiesen. Hierzu wurden Antikörper gegen basisches Myelinprotein (Boehringer Nr. 1118099) oder Proteolipid Protein (Ian Griffiths, Department of Veterinary Clinical Studies, University of Glasgow, Bearsden, Scotland) verwendet. Die Schnitte wurden anschließend einer DNA Hybridisierung mit einem DNA-Sondenmolekül für Maus-Satelliten-DNA (Hörz & Altenburger, Nucl. Acids Res. 9:683-696, 1981) unterzogen (Brüstie et al., Neuron 15:1275-1285, 1995). Diese Doppelmarkierung gestattete es, gebildetes Myelin eindeutig den transplantierten Zellen zuzuordnen. In solchen Experimenten ließ sich eine Einwanderung der in den Ventrikel implantierten Zellen in eine Vielzahl tel-, di- und mesencephaler Hirnregionen nachweisen (14 untersuchte Tiere). Hybridisierte Zellen fanden sich unter anderem in Cortex, Hippocampus, Septum, Striatum, Bulbus olfactorius, Thalamus, Hypothalamus, Tectum, Cerebellum, Corpus callosum, in der vorderen Commissur sowie im Tractus opticus und innerhalb des Sehnerven. Nach Transplantation ließen sich im Ventrikel system der Empfängertiere keine raumfordernden Ansammlungen von Donorzellen nachweisen. Von 35 operierten Embryonen zeigten 11 männliche Nachkommen die Symptome einer Myelindefizienz. Von diesen waren 8 erfolgreich intraventrikulär transplantiert worden. In 6 dieser 8 Tiere konnte eine von den Donorzellen ausgehende Myelinisierung nachgewiesen werden. Hierzu wurde eine Doppelmarkierung hybridisierter Zellen mit dem PLP- oder MBP-Antikörper verwendet. In den verbleibenden 2 Fällen war die Zahl der inkorporierten Zellen für eine Doppelmarkierung zu niedrig; ein Screening mit dem Maus-spezifischen Antikörper M2 (Zhou et al., J. Comp. Neurol. 292:320-330, 1990) zeigte jedoch auch in diesen Tieren in das Wirtsgewebe inkorporierte Gliazellen. Die Donorzell-vermittelte Myelinisierung war am ausgeprägtesten in Faserbahnen wie z.B. dem Corpus callosum, der vorderen Commissur und commissuralen Fasern des Tectums. Darüber hinaus waren jedoch auch an zahlreichen Stellen innerhalb der grauen Substanz myelinisierende Donorzellen nachweisbar, so z.B. in Cortex, Septum, Thalamus, Hypothalamus und Tectum. In 7 der 8 erfolgreich transplantierten myelindefizienten Tiere liessen sich auch von den ES-Zellen abstammende inkorporierte Astrozyten nachweisen. Diese wurden immunhistochemisch mit Antikörpern gegen die Maus-spezifischen Antigene M2 (Zhou et al., J. Comp. Neurol. 292:320-330, 1990) und M6 (Lund et al., Neurosci. Lett. 61:221-226, 1985) oder durch eine immunhistochemische Doppelmarkierung von mit der Maus-spezifischen DNA Probe markierten Zellen mit einem Antikörper gegen saures Gliafaserprotein (GFAP; ICN Nr. 69-110) nachgewiesen.

### Beispiel 5: Transplantation neuraler Sphäroide

### 5.1. Transplantation neuraler Sphäroide im Ibotensäure-Läsionsmodell der Ratte

Um eine anatomische und funktionelle Rekonstitution neuronaler Zellen durch die verfahrensgemäß hergestellten neuralen Vorläuferzellen zu überprüfen, wurden gemäß Beispiel 3.1 aus der ES Zellinie J1 gewonnene, 5 Tage alte neurale Sphäroide in das Striatum von erwachsenen Sprague-Dawley Ratten implantiert, in denen zuvor durch stereotaktische Injektion des Neurotoxins Ibotensäure ein einseitiger Neuronenuntergang im Striatum induziert worden war. Die striatale Ibotensäure-Läsion ist Stand der Technik und das verwendete Läsionsprotokoll veröffentlicht (Brüstle et al., aus: Current Protocols in Neuroscience, Unit 3.10, John Wiley, New York, 1997). Für die Transplantation wurden das Medium und die darin schwimmenden Sphäroide der Zellkulturschale entnommen und in ein 50 ml Plastikröhrchen überführt. Nach Zugabe von DNase zu einer Gesamtkonzentration von 0.1% wurden die Späroide bei 150 × g über 5 min bei RT sedimentiert und dann in CMF-HBSS, welche 2 g/L Glucose (Sigma Nr. G-7021) enthielt, in einem 0.5 ml Plastikreaktionsröhrchen resuspendiert. Die so resuspendierten Sphäroide wurden bis zur Transplantation (bis zu 4 Stunden) auf Eis gelagert. Die Empfangertiere wurden in üblicher Weise betäubt und die Sphäroide über eine stereotaktische Injektion in das Striatum eingeführt. Für die Injektion wurde eine Glaskapillare mit einer Öffnung von etwa 0.25 - 0.75 mm verwendet. Das verwendete Transplantationsprotokoll ist Stand der Technik und ausführlich publiziert (Brüstle et al., aus: Current Protocols in Neuroscience, Unit 3.10, John Wiley, New York, 1997). Es wurden insgesamt 6 Tiere transplantiert. Dabei wurden die sich am Boden des 0.5 ml Röhrchens angesammelten Sphäroide in die Glaskapillare aufgezogen und mit Hilfe eines stereotaktischen Rahmens (Stoelting Nr. 51600) in das Striatum injiziert. Die Sphäroide wurden dabei über 2 (n=3) oder 5 (n=3) Stellen innerhalb des geschädigten Striatums verteilt. Hierbei wurden folgende stereotaktischen Daten verwendet:

### Für 2 Implantationsorte:

| | | |
|---|---|---|
| Ort 1: | Frontzahnhalter: | - 2.3 mm |
| | Anteroposteriore Ausrichtung: | + 0.2 mm (relativ zum Bregma) |
| | Mediolaterale Ausrichtung: | 3.0 mm (relativ zu Sagittalnaht) |
| | Injektionstiefe | 5.5 mm (relativ zur Dura) |
| Ort 2: | Frontzahnhalter: | - 2.3 mm |
| | Anteroposteriore Ausrichtung: | + 0.2 mm (relativ zum Bregma) |
| | Mediolaterale Ausrichtung: | 3.0 mm (relativ zu Sagittalnaht) |
| | Injektionstiefe | 4.0 mm (relativ zur Dura) |

### Für 5 Implantationsorte:

| | | |
|---|---|---|
| Ort 1: | Frontzahnhalter: | - 2.3 mm |
| | Anteroposteriore Ausrichtung: | + 0.2 mm (relativ zum Bregma) |
| | Mediolaterale Ausrichtung: | 3.0 mm (relativ zu Sagittalnaht) |
| | Injektionstiefe | 6.5 mm (relativ zur Dura) |
| Ort 2: | Frontzahnhalter: | - 2.3 mm |
| | Anteroposteriore Ausrichtung: | + 0.2 mm (relativ zum Bregma) |
| | Mediolaterale Ausrichtung: | 3.0 mm (relativ zu Sagittalnaht) |
| | Injektionstiefe | 5.3 mm (relativ zur Dura) |
| Ort 3: | Frontzahnhalter: | - 2.3 mm |
| | Anteroposteriore Ausrichtung: | + 0.2 mm (relativ zum Bregma) |
| | Mediolaterale Ausrichtung: | 3.0 mm (relativ zu Sagittalnaht) |
| | Injektionstiefe | 4.0 mm (relativ zur Dura) |
| Ort 4: | Frontzahnhalter: | - 2.3 mm |
| | Anteroposteriore Ausrichtung: | + 1.5 mm (relativ zum Bregma) |
| | Mediolaterale Ausrichtung: | 2.5 mm (relativ zu Sagittalnaht) |
| | Injektionstiefe | 6.0 mm (relativ zur Dura) |
| Ort 5: | Frontzahnhalter: | - 2.3 mm |
| | Anteroposteriore Ausrichtung: | + 1.5 mm (relativ zum Bregma) |
| | Mediolaterale Ausrichtung: | 2.5 mm (relativ zu Sagittalnaht) |
| | Injektionstiefe | 4.5 mm (relativ zur Dura) |

Pro Injektionsort wurden 10 µl der Sphäroid Suspension implantiert.

Da es sich um Xenotransplantate handelte, wurden die Tiere einer Immunsuppression unterzogen. Hierzu erhielten die Empfängertiere täglich, beginnend 1 Tag vor Transplantation, eine intraperitoneale Injektion von 20 mg Cyclosporin A pro kg Körpergewicht (Sandimmun, Fa. Sandoz). Um opportunistische Infektionen zu vermeiden, wurde dem Trinkwasser während der Immunsuppressionsbehandlung Tetracyclin zugesetzt (Achromycin, Fa. Lederle; Endkonzentration 100 mg/L).

### 5.2. Analyse der funktionellen Verbesserung der Transplantatempfänger

Von den 6 operierten Tieren überlebten 4 Tiere mehr als 4 Wochen nach Transplantation. Bei diesen Tieren ergab die Untersuchung des Amphetamin-induzierten Rotationsverhaltens vor und nach Transplantation folgende Werte (Mittelwert der Rotationen pro Minute):

| | | | |
|---|---|---|---|
| Tier 200: | 34 Tage vor Transplantation: | | 9.7 |
| | 37 Tage nach Transplantation: | 13.9 | |
| | | | |
| Tier 201: | 24 Tage vor Transplantation: | | 13.5 |
| | 37 Tage nach Transplantation: | 1.3 | |
| | | | |
| Tier 204: | 43 Tage vor Transplantation: | | 12.7 |
| | 50 Tage nach Transplantation: | 0.5 | |
| | | | |
| Tier 205: | 86 Tage vor Transplantation: | | 9.0 |
| | 42 Tage nach Transplantation: | 1.0 | |

Zur Messung des Rotationsverhaltens wurde den Tieren intraperitoneal eine Dosis von 5 mg D-Amphetamin Sulfat (Sigma Nr. A-3278) pro kg Körpergewicht injiziert. Nach 5-minütiger Einwirkungszeit wurde die Zahl der ausgelösten Rotationen pro Minute über einen Zeitraum von 90 Minuten gemessen und gemittelt. Die Messung des Amphetamin-induzierten Rotationsverhalten als Parameter für die Funktion striataler Transplantate ist Stand der Technik und publiziert (Brüstle et al., aus: Current Protocols in Neuroscience, Unit 3.10, John Wiley, New York, 1997). Die Daten zeigen, daß 3 der 4 untersuchten Tiere eine deutliche Reduktion des Rotationsverhaltens aufwiesen.

### 5.3. Histologische Analyse der Transplantatempfänger

Für die histologische Untersuchung der Transplantate wurden die Tiere 5-9 Wochen nach Transplantation betäubt und durch eine transkardiale Perfusion mit einer 4%igen Paraformaldehydlösung in PBS in üblicher Weise fixiert. Die Gehirne wurden in üblicher Weise präpariert und über Nacht in der Fixationslösung bei 4°C nachfixiert. Im Anschluß daran wurden auf einem Vibratom 50 µm Schnitte angefertigt. Die Donorzellen wurden mittels DNA *in situ* Hybridisierung mit einer Probe für Maus Satelliten DNA nachgewiesen (Hörz & Altenburger, Nucl. Acids Res. 9:683-696, 1981; Brüstle et al., Neuron 15:1275-1285, 1995). Dabei fanden sich in allen 4 der in den Verhaltenstests (Beispiel 5.2) untersuchten Tiere hybridisierte Zellen im Implantationsgebiet. Ein Teil der hybridisierten Zellen war zudem aus dem Implantationsgebiet in benachbarte Hirnabschnitte, besonders in das Corpus Callosum, eingewandert. Das Tier 200 wies einen sehr weiten Seitenventrikel und ein intraventrikulär lokalisiertes Transplantat auf. Offenbar war durch die vorausgegangene Ibotensäureläsion eine starke Atrophie des Striatums mit konsekutiver Ventrikelerweiterung entstanden und die nachfolgend implantierten Zellen landeten anstatt im Striatum im Seitenventrikel. Die inkorrekte Lage im Seitenventrikel erklärt auch die ausbleibende funktionelle Verbesserung dieses Empfängertieres in den Rotationstests.

Neben positivem Hybridisierungssignal wiesen die transplantierten Zellen eine deutliche Expression des Maus-spezifischen neuralen Antigens M6 auf. Dieses Antigen wird besonders auf Axonen exprimiert (Lund et al., Neurosci. Lett. 61:221-226, 1985; Antikörper von C. Lagenaur, Department of Neurobiology, University of Pittsburgh School of Medicine, 818A Scaife Hall, Pittsburgh, Pennsylvania 15261, USA, Verdünnung 1:10). In den Empfangertieren ließen sich zahlreiche M6-positive Axone erkennen, welche vom Transplantat ausgingen und in das umgebende Hirngewebe eingesproßt waren. Dies weist darauf hin, daß die aus den transplantierten Sphäroiden hervorgegangenen Nervenzellen das Empfängergehim innervieren.

## Patentansprüche

1. Isolierte, nach Transplantation in das Nervensystem nicht-tumorigene gereinigte Vorläuferzellen aus embryonalen Stammzellen mit neuronalen oder glialen Eigenschaften.

2. Isolierte, gereinigte nicht-tumorigene Vorläuferzellen mit neuronalen oder glialen Eigenschaften aus embryonalen Stammzellen, enthaltend höchstens etwa 15% primitive embryonale und nicht-neurale Zellen, erhältlich durch folgende Schritte:
a) proliferieren von ES-Zellen,
b) kultivieren der ES-Zellen aus Schritt a) zu neuralen Vorläuferzellen,
c) proliferieren der neuralen Vorläuferzellen in einem Wachstumsfaktor-haltigen serumfreien Medium,
d) proliferieren der neuralen Vorläuferzellen aus Schritt c) in einem weiteren Wachstumsfaktor-haltigen serumfreien Medium und isolieren der gereinigten neuralen Vorläuferzellen und
e) proliferieren der neuralen Vorläuferzellen aus Schritt d) in einem weiteren Wachstumsfaktor-haltigen serumfreien Medium und isolieren der gereinigten Vorläuferzellen mit neuronalen oder glialen Eigenschaften.

3. Zellen nach Anspruch 2, wobei die ES-Zellen in Schritt a) zu Zellaggregaten, insbesondere Embryoid Bodies proliferiert werden.

4. Isolierte, gereinigte nicht-tumorigene Vorläuferzellen mit neuronalen oder glialen Eigenschaften aus embryonalen Stammzellen, enthaltend höchstens etwa 15% primitive embryonale und nicht-neurale Zellen, erhältlich durch folgende Schritte:
a') proliferieren von ES-Zellen,
b') kultivieren der ES-Zellen aus Schritt a') zu neuralen Vorläuferzellen,
c') proliferieren der neuralen Vorläuferzellen in einem Wachstumsfaktor-haltigen serumfreien Medium,
d') proliferieren der neuralen Vorläuferzellen aus Schritt c') in einem weiteren Wachstumsfaktor-haltigen serumfreien Medium zu neuralen Sphäroiden und isolieren der neuralen Sphäroide und
e') proliferieren der neuralen Sphäroide aus Schritt d') in einem Wachstumsfaktor-haltigen serumfreien Medium bis zur Ausbildung eines aus glialen Vorläuferzellen bestehenden Zellrasens und isolieren der gereinigten Vorläuferzellen mit glialen Eigenschaften.

5. Zellen nach Anspruch 4, wobei die ES-Zellen in Schritt a') zu Zellaggregaten, insbesondere Embryoid Bodies proliferiert werden.

6. Zellen nach einem der Ansprüche 1 bis 5 in Form eines Zellrasens.

7. Zellen nach einem der Ansprüche 1 bis 5 in Form von Sphäroiden.

8. Zellen nach einem der Ansprüche 1 bis 5, umfassend Zellen mit neuronalen, astrozytären und/oder oligodendroglialen Eigenschaften.

9. Zellen nach einem der Ansprüche 1 bis 5, wobei die embryonalen Stammzellen aus Oocyten nach einer Kerntransplantation erhalten worden sind.

10. Zellen nach einem der Ansprüche 1 bis 5, wobei die embryonalen Stammzellen aus embryonalen Keimzellen erhalten worden sind.

11. Zellen nach einem der Ansprüche 1 bis 5, wobei die Zellen Säugerzellen sind.

12. Zellen nach Anspruch 11, wobei die Zellen aus der Gruppe umfassend Maus, Ratte, Hamster, Schaf, Schwein, Rind, Primaten oder Mensch isoliert worden sind.

13. Zellen nach einem der Ansprüche 1 bis 10, wobei die Zellen gentechnisch modifiziert worden sind.

14. Zellen nach einem der Ansprüche 1 bis 13 in tiefgefrorener Form.

15. Zellbibliothek, umfassend autologe und nicht autologe Zellen nach einem der Ansprüche 1 bis 14.

16. Sphäroide, umfassend differenzierte neurale Zellen der Vorläuferzellen nach einem der Ansprüche 1 bis 5.

17. Sphäroide nach Anspruch 16, **dadurch** erhalten, daß die Vorläuferzellen nach einem der Ansprüche 1 bis 5 *in vitro* zur Differenzierung induziert worden sind.

18. Verfahren zur Herstellung von gereinigten Vorläuferzellen mit neuronalen oder glialen Eigenschaften, umfassend die folgenden Schritte:
a) proliferieren von ES-Zellen,
b) kultivieren der ES-Zellen aus Schritt a) zu neuralen Vorläuferzellen,
c) proliferieren der neuralen Vorläuferzellen in einem Wachstumsfaktor-haltigen serumfteien Medium,
d) proliferieren der neuralen Vorläuferzellen aus Schritt c) in einem weiteren Wachstumsfaktor-haltigen serumfreien Medium und isolieren der gereinigten neuralen Vorläuferzellen und
e) proliferieren der neuralen Vorläuferzellen aus Schritt d) in einem weiteren Wachstumsfaktor-haltigen serumfreien Medium und isolieren der gereinigten Vorläuferzellen mit neuronalen oder glialen Eigenschaften.

19. Verfahren nach Anspruch 18. wobei die ES-Zellen in Schritt a) zu Zellaggregaten, insbesondere Embryoid Bodies proliferiert werden.

20. Verfahren nach Anspruch 18 oder 19, wobei das Wachstumsfaktor-haltige serumfreie Medium in Schritt c) bFGF umfaßt.

21. Verfahren nach einem der Ansprüche 18 bis 20, wobei das Wachstumsfaktor-haltige serumfreie Medium in Schritt d) bFGF und EGF umfaßt.

22. Verfahren nach einem der Ansprüche 18 bis 21, wobei das Wachstumsfaktor-haltige serumfreie Medium in Schritt e) bFGF und PDGF umfaßt.

23. Verfahren zur Herstellung von gereinigten Vorläuferzellen mit neuronalen oder glialen Eigenschaften, umfassend die folgenden Schritte:
a') proliferieren von ES-Zellen,
b') kultivieren der ES-Zellen aus Schritt a') zu neuralen Vorläuferzellen,
c') proliferieren der neuralen Vorläuferzellen in einem Wachstumsfaktor-haltigen serumfreien Medium,
d') proliferieren der neuralen Vorläuferzellen aus Schritt c') in einem weiteren Wachstumsfaktor-haltigen serumfreien Medium zu Sphäroiden mit neuronalen und
glialen Differenzierungspotential und isolieren der Sphäroide und
e') proliferieren der neuralen Sphäroide aus Schritt d') in einem Wachstumsfaktor-haltigen serumfreien Medium bis zur Ausbildung eines aus glialen Vorläuferzellen bestehenden Zellrasens und isolieren der gereinigten Vorläuferzellen mit glialen Eigenschaften.

24. Verfahren nach Anspruch 23, wobei die ES-Zellen in Schritt a') zu Zellaggregaten, insbesondere Embryoid Bodies proliferiert werden.

25. Verfahren nach Anspruch 23 oder 24 ferner umfassend den Schritt:
f) Steuerung der Differenzierung der glialen Vorläuferzellen aus Schritt e') in eine astrozytäre oder in eine oligodendrogliale Richtung und isolieren der Vorläuferzellen mit astrozytären oder oligodendroglialen Eigenschaften.

26. Verfahren nach einem der Ansprüche 23 bis 25, wobei das Wachstumsfaktor-haltige serumfreie Medium in Schritt c') bFGF umfaßt.

27. Verfahren nach einem der Ansprüche 23 bis 26, wobei das Wachstumsfaktor-haltige serumfreie Medium in Schritt d'), e') und f) bFGF und/oder EGF umfaßt.

28. Verfahren nach einem der Ansprüche 25 bis 27, wobei das Medium in Schritt f') entweder CNTF oder T3 umfaßt.

29. Verfahren nach einem der Ansprüche 18 bis 28, wobei das Verfahren mit Zelltrennungs- und Sortierverfahren kombiniert wird.

30. Verfahren nach einem der Ansprüche 18 bis 29, wobei man die gereinigten Vorläuferzellen in einem Injektionsmedium aufnimmt.

31. Verwendung der Vorläuferzellen nach einem der Ansprüche 1 bis 15 zur Transplantation in das Nervensystem.

32. Verwendung der Sphäroide nach Anspruch 16 oder 17 zur Transplantation in das Nervensystem.

33. Verwendung der Vorläuferzellen nach einem der Ansprüche 1 bis 15 zur Therapie von neuralen Defekten.

34. Verwendung der Vorläuferzellen nach einem der Ansprüche 1 bis 15 zur Rekonstitution neuronaler Zellen.

35. Verwendung der Vorläuferzellen nach einem der Ansprüche 1 bis 15 zur Remyelinisierung demyelinisierter Bereiche des Nervensystems.

36. Verwendung der Vorläuferzellen nach einem der Ansprüche 1 bis 15 für zellvermittelten Gentransfer ins Nervensystem.

37. Verwendung der Vorläuferzellen nach einem der Ansprüche 1 bis 15 zur *in vitro* Gewinnung von Polypeptiden.

38. Arzneimittelzusammensetzung, enthaltend Vorläuferzellen nach einem der Ansprüche 1 bis 15 zur Therapie von neuralen Defekten.
